Europäisches Patentamt

European Patent Office

Office européen des brevets

(11) Veröffentlichungsnummer: **0 214 548 B1**

# (12) EUROPÄISCHE PATENTSCHRIFT

(45) Veröffentlichungstag der Patentschrift: **27.12.91**

(51) Int. Cl.⁵: **C12N 15/61**, C12N 9/90

(21) Anmeldenummer: 86111810.7

(22) Anmeldetag: 26.08.86

(54) **DNA-Sequenzen, rekombinante DNA-Moleküle und Verfahren zur Herstellung des Enzyms Mutarotase aus Acinetobacter calcoaceticus.**

(30) Priorität: **03.09.85 DE 3531360**

(43) Veröffentlichungstag der Anmeldung:
**18.03.87 Patentblatt 87/12**

(45) Bekanntmachung des Hinweises auf die
Patenterteilung:
**27.12.91 Patentblatt 91/52**

(84) Benannte Vertragsstaaten:
**BE CH DE FR GB IT LI NL SE**

(56) Entgegenhaltungen:
**EP-A- 0 173 919**
**WO-A-85/01961**
**FR-A- 2 201 768**

**CHEMICAL ABSTRACTS, Band 98, 17. Januar
1983, Seite 414, Zusammenfassung Nr.
15489p, Columbus, Ohio, US; & JP-A-57 152
885 (TOYOBO CO., LTD.) 21.09.1982**

**idem**

(73) Patentinhaber: **MERCK PATENT GESELL-
SCHAFT MIT BESCHRÄNKTER HAFTUNG
Frankfurter Strasse 250 Postfach 4119
W-6100 Darmstadt(DE)**

(72) Erfinder: **Gatz, Christiane, Dr.
Höchster Strasse 4
W-4370 Marl(DE)**
Erfinder: **Altschmied, Joachim
Wertheimer Strasse 27
W-8000 München 60(DE)**
Erfinder: **Gassen, Hans-Günther, Prof. Dr.
Flachsbachweg 54
W-6100 Darmstadt(DE)**
Erfinder: **Hillen, Wolfgang, Prof. Dr.
Eichenweg 17
W-8520 Erlangen(DE)**

CHEMICAL ABSTRACTS, Band 82, 23. Juni 1975, Seite 250, Zusammenfassung Nr. 167297g, Columbus, Ohio, US; P. SAMMLER et al.: "Mutarotase in galactose-induced baker's yeast" & FOLIA MICROBIOL. (PRAGUE) 1974, 19(6), 479-88

idem

CHEMICAL ABSTRACTS, Band 79, 17. September 1973, Seite 189, Zusammenfassung Nr. 63239m, Columbus, Ohio, US; S.A. MULHERN et al.: "Physical characteristics and chemiosmotic transformations of mutarotases from various species" & J. BIOL: CHEM. 1973, 248(12), 4163-73

idem

CHEMICAL ABSTRACTS, Band 76, 28. Februar 1972, Seite 162, Zusammenfassung Nr. 43405x, Columbus, Ohio, US; F. HUCHO et al.: "Enzymically catalyzed mutarotation. Mechanism of action of mutarotase (aldose 1-epimerase) from Escherichia coli" & EUR. J. BIOCHEM. 1971, 23(3), 489-96

idem

CHEMICAL ABSTRACTS, Band 68, 20. Mai 1968, Seite 8972, Zusammenfassung Nr. 93109j, Columbus, Ohio, US; W. SACKS: "Isolation and properties of mutarotase in erythrocytes" & ARCH. BIOCHEM. BIOPHYS. 123(3), 507-13(1968)

idem

CHEMICAL ABSTRACTS, Band 67, 20. November 1967, Seite 9127, Zusammenfassung Nr. 97129t, Columbus, Ohio, US; J.M. BAILEY et al.: "Mutarotases. I. Purification and properties of a mutarotase from higher plants" & J. BIOL. CHEM. 242(18), 4263-9(1967)

idem

BIOCHEMISTRY, Band 12, Nr. 13, 1973, Seiten 2490-2495; P.H. FISHMAN et al.: "Analysis of a crystalline mutarotase from bovine kidney cortex"

idem

NUCLEIC ACIDS RESEARCH, Band 14, Nr. 10, 1986, Seiten 4309-4323, IRL Press Ltd., Oxford, GB; C. GATZ et al.: "Acinetobacter calcoaceticus encoded mutarotase: nucleotide sequence analysis of the gene and characterization of its secretion in Escherichia coli"

**Beschreibung**

Die Erfindung betrifft die Bereitstellung einer DNA-Sequenz, die für ein Polypeptid mit der biologischen Aktivität des Enzyms Mutarotase codiert.

Außerdem betrifft die Erfindung rekombinante DNA-Moleküle,d.h. Clonierungs- und Expressions-Vektoren, zur Verwendung bei der Herstellung eines Polypeptids mit der biologischen Aktivität des Enzyms Mutarotase sowie mit solchen Vektoren transformierte Wirtsorganismen, beispielsweise Bakterien, Hefen, andere Pilze, tierische oder menschliche Zellen.

Schließlich betrifft die Erfindung die Verwendung des genannten Polypeptids mit der biologischen Aktivität des Enzyms Mutarotase bei der Beschleunigung enzymatischer Nachweisreaktionen oder Umsetzungen von Aldosen.

Mutarotase (Aldose-1-epimerase, EC 5.1.3.3) beschleunigt bekanntlich die Gleichgewichtseinstellung zwischen den $\alpha$- und $\beta$-Anomeren von Aldohexosen, z.B. zwischen $\alpha$- und $\beta$-Glucose oder $\alpha$- und $\beta$-Galactose. Die Hauptanwendung des Enzyms liegt in der analytischen Biochemie bei der Beschleunigung enzymatischer Nachweisreaktionen für Aldosen mit Hilfe von für die $\alpha$- oder $\beta$-Form spezifischen Enzymen, bei denen die Gleichgewichtseinstellung zwischen beiden Anomeren der geschwindigkeitsbestimmende Schritt ist, z.B. bei Bestimmungsmethoden mit Glucosedehydrogenase, Glucoseoxidase oder Galactosedehydrogenase.

Eine großtechnische Anwendung wäre z.B. für den Glucoamylase/Glucoseisomerase-Prozeß interessant, weil das Enzym Glucoamylase $\beta$-Glucose freisetzt, die erst nach Mutarotation zur $\alpha$-Form von der Glucoseisomerase umgesetzt werden kann.

Mutarotase ist in der Natur weit verbreitet, sie kommt in verschiedenen Mikroorganismen (Bakterien, Hefen, Fadenpilzen), in Pflanzen und in tierischen Geweben vor.

Nennenswerte Enzymgehalte, die eine Isolierung von Mutarotase im technischen Maßstab ermöglichen, wurden bisher nur in Nieren von Säugetieren (Rind, Schwein) gefunden; alle bekannten Handelsprodukte werden aus Nieren hergestellt. Aus Bailey, Meth. Enzymol. 1975, 478, ist bekannt, daß Rindernieren mehr als die 60fache Mutarotase-Aktivität pro g Frischgewicht enthalten als z.B. Escherichia coli. In Biochim. Biophys. Acta 662, 285 (1981) wird erstmals ein Verfahren zur mikrobiologischen Herstellung von Mutarotase aus Stämmen von Aspergillus niger beschrieben. Danach wurde aus dem besten Stamm eine Mutarotase-Aktivität von 4,4 mU/ml Kulturbrühe erzielt, wobei die Michaelis-Konstante 50 mM betrug und das pH-Optimum im Bereich von 5 bis 7 lag.

Mit Hilfe des genannten mikrobiologischen Verfahrens kann jedoch das Enzym Mutarotase nur in niedrigen Ausbeuten im Vergleich zum Herstellungsverfahren aus Rindernieren gewonnen werden. Außerdem sind die Eigenschaften des Enzyms aus Aspergillus niger für die Gleichgewichtseinstellung im Rahmen enzymatischer Bestimmungen von Aldosen ungünstig.

Der Erfindung liegt somit die Aufgabe zugrunde, ein Polypeptid mit der biologischen Aktivität des Enzyms Mutarotase bereitzustellen und ein gentechnologisches Verfahren zu schaffen, das die technische Herstellung dieses Proteins in großen Mengen ermöglicht.

Der Ausdruck "Polypeptid mit der biologischen Aktivität des Enzyms Mutarotase" bezeichnet ein Polypeptid bzw. Protein, dessen Aminosäure-Sequenz der nativen Mutarotase aus Acinetobacter calcoaceticus entspricht, ähnlich zu dieser Aminosäure-Sequenz ist oder nur einen Teilbereich davon umfaßt. Erfindungsgemäß werden auch entsprechende Fusionsproteine als "Polypeptide mit der biologischen Aktivität des Enzyms Mutarotase" bezeichnet.

Als Mutarotase-bildende Spezies dienen vorzugsweise Mikroorganismen der Gattung Acinetobacter. Hiervon werden diejenigen der Stämme Aceinetobacter calcoaceticus DSM 30007, DSM 30008, DSM 30010 oder DSM 30011, insbesondere DSM 30008, bevorzugt sowie deren Mutanten oder Varianten, in die Teile der entsprechenden genetischen Information eingebracht wurden.

Als Wirtsorganismen, in die Mutarotase codierende DNA transplantiert werden kann, kommen in erster Linie Mikroorganismen, aber auch pflanzliche, tierische oder menschliche Zellen in Frage. Bevorzugt sind Mikroorganismen wie Bakterien, Hefen und Fadenpilze, insbesondere E.coli-Bakterien.

Zur Bereitstellung eines gentechnologischen Verfahrens zur Herstellung eines Polypeptids mit der biologischen Aktivität des Enzyms Mutarotase werden erfindungsgemäß folgende Schritte ausgeführt:

Zunächst wird beispielsweise der Mikroorganismus Acinetobacter calcoaceticus Stamm Nr. DSM 30008 in einem geeigneten Kulturmedium kultiviert. Anschließend wird aus dieser Kultur die native Mutarotase des Mikroorganismus isoliert und proteinbiochemisch analysiert. Nach der Feststellung von Aminosäure-Teilsequenzen der genannten Mutarotase werden den Aminosäuren-Positionen 12 bis 17 und 353 bis 359 des Mutarotase-Polypeptids entsprechende Oligonucleotide synthetisiert. Aufgrund der Degenerierung des genetischen Codes gibt es dabei für die Sequenz der den Aminosäure-Positionen entsprechenden Oligodesoxynucleotide jeweils verschiedene Möglichkeiten. Erfindungsgemäß werden deshalb jeweils mehrere verschiedene Oligodesoxynucleotide synthetisiert und bei den nachfolgenden Hybridisierungen als Oligonucleotidgemische I und II verwendet (vgl. Tabelle III).

In einem weiteren Arbeitsgang wird aus in einem geeigneten Kulturmedium gezüchteten Acinetobacter calcoaceticus-Mikroorganismen (Stamm Nr. 30008) chromosomale DNA isoliert. Die chromosomale DNA wird dann mit den Restriktionsendonucleasen Bcl I, EcoRI und Hind III gespaltem, auf Nitrocellulose geblottet und mit den Oligonucleotidgemischen I und II hybridisiert. Dabei hybridisiert mit dem Oligonucleotidgemisch I ein 6400 bp großes Bcl I-DNA-Fragment. Dieses Fragment wird dann in die BamHI-Spaltstelle des Plasmids pBR327 cloniert. Die Restriktionsanalyse in Verbindung mit der Bestimmung einer Teilsequenz des clonierten BclI-Fragments ergibt, daß dieses Fragment für den N-terminalen Bereich des Mutarotase-Polypeptids codiert.

Mit dem Oligonucleotidgemisch II hybridisiert ein etwa 1500 bp großes Hind III-Fragment der chromosomalen DNA von Acinetobacter calcoaceticus (Stamm Nr. 30008). Dieses Fragment wird daher in die Hind III-Spaltstelle des Bakteriophagen M13mp11 cloniert. Die anschließende Kartierung und Bestimmung einer Teilnucleotid-Sequenz zeigt, daß das clonierte Hind III-Fragment für den C-terminalen Bereich des Mutarotase-Polypeptids codiert.

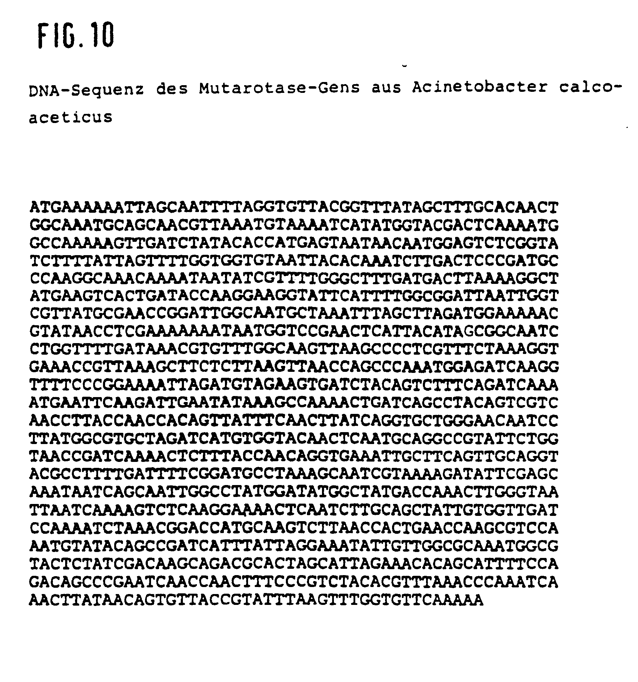

Gegenstand der Erfindung ist somit die DNA-Sequenz gemäß Figur 10, die für ein Polypeptid mit der biologischen Aktivität des Enzyms Mutarotase codiert.

Gegenstand der Erfindung ist ferner ein rekombinantes Expressionsplasmid, in dem der gesamte codierende Bereich des Mutarotase-Gens unter Kontrolle des $\lambda$-$P_L$-Promotors steht und der die gesamte DNA-Sequenz des Mutarotase-Gens bestimmt. Das für den N-terminalen Bereich des Mutarotase-Polypeptids codierende DNA-Fragment wird dabei aus dem vorstehend genannten rekombinanten pBR327-Clon abgeleitet, das für den C-terminalen Bereich des Mutarotase-Polypeptids codierende DNA-Fragment aus dem vorstehend genannten M13mp11-Clon. Erfindungsgemäß können anstelle des $\lambda$-$P_L$-Promotors als Expressions-Kontroll-Sequenz ebenso ein E.coli Promotor-System wie das E.coli lac-System, das E.coli $\beta$-Lactamasesystem, das E.coli trp-System oder der E.coli Lipoprotein-Promotor, eine Hefeexpressions-Kontroll-Sequenz oder eine andere eukaryontische Expressions-Kontroll-Sequenz verwendet werden. Wichtig ist dabei nur die funktionelle Verknüpfung des Gens mit der Expressions-Kontroll-Sequenz sowie die Auswahl einer geeigneten Expressions-Kontroll-Sequenz für einen bestimmten Wirtsorganismus.

Gegenstand der Erfindung ist insbesondere das Expressionsplasmid pWH 1372 , welches die das Mutarotase-Polypetid codierende erfindungsgemäße DNA-Sequenz enthält.

Nach der Konstruktion des rekombinanten Expressionsvektors wird dieser in üblicher Weise in einen transformierbaren Wirtsorganismus, z.B. ein E.coli-Bakterium, wie z.B. E.coli Stamm Nr. 69 (E.coli K12 $\Delta$ H1) eingebracht.

Anschließend wird der transformierte Wirtsorganismus in an sich bekannter Weise in einem geeigneten Nährmedium kultiviert und das bei der Expression gebildete Polypeptid mit der biologischen Aktivität des Enzyms Mutarotase wird daraus nach Standardmethoden gewonnen. Gegenstand der Erfindung ist somit auch der die erfindungsgemäße DNA enthaltende Wirtsorganismus, insbesondere E.coli WH1372, in welchen das Plasmid $p^{WH}1372$ transformiert wurde.

Erfindungsgemäß kann unter Verwendung der isolierten, Mutarotase-codierenden DNA-Sequenz aus Acinetobacter calcoaceticus als Sondenmolekül in Genbanken Mutarotasebildender Mikroorganismen oder Säuger das entsprechende Mutarotase-Gen identifiziert und aus diesen Genbanken isoliert werden. Durch funktionelle Verbindung der Mutarotase-codierenden Sequenzen dieser Organismen mit geeigneten Expressions-Kontroll-Sequenzen ist auf diese Weise auch die Herstellung von Polypeptiden mit der biologischen Aktivität des Enzyms Mutarotase von Säugern, wie Rind und Schwein, und Mikroorganismen, z.B. der Gattung Aspergillus, in beliebiger Menge möglich. Das nach dem erfindungsgemäßen Verfahren hergestellte Polypeptid mit der biologischen Aktivität des Enzyms Mutarotase ist beispielsweise durch folgende Eigenschaften charakterisiert. Das Molekulargewicht beträgt etwa 40.000. Das pH-Aktivitätsoptimum liegt im pH-Bereich von 7 bis 8, das Temperaturoptimum bei 42 $^\circ$C bis 46 $^\circ$C. Die Michaelis-Konstante für Glucose beträgt 6 bis 8 mM.

Bei Konzentrationen von 2 mM hemmen $Hg^{++}$- und $Fe^{+++}$-Ionen die Aktivität etwa zur Hälfte; $Cu^{++}$-, $Co^{+++}$- und $Zn^{++}$-Ionen hemmen in gleicher Konzentration vollständig.

Das erfindungsgemäß hergestellte Polypeptid mit der biologischen Aktivität des Enzyms Mutarotase ist spezifisch für Aldohexosen. Die Aktivitätsbestimmung wird wie folgt durchgeführt:

$$\alpha\text{-Glucose} \xrightleftharpoons{\text{Mutarotase}} \beta\text{-Glucose}$$

$$\beta\text{-Glucose} + \text{NAD} \xrightarrow{\text{Glucosedehydrogenase}} \text{Gluconolacton} + \text{NADH}_2$$

Man läßt Mutarotase, Glucosedehydrogenase und NAD mit einer frisch angesetzten Lösung von $\alpha$-Glucose reagieren und bestimmt das gebildete NADH$_2$ durch Extinktionsmessung bei 366 nm. Die Messung der Beschleunigung der NADH$_2$-Bildung durch Mutarotase im Verhältnis zu einem Vergleichswert gestattet die Berechnung der Mutarotase-Aktivität der eingesetzten Lösung.

Die Bestimmung der Mutarotase-Aktivität erfolgt also in an sich bekannter Weise über eine Glucosedehydrogenasebestimmung. Letztere zählt zu den Standardmethoden beispielsweise in der klinischen Chemie.

Die Enzymaktivität der Mutarotase ergibt sich aus der Differenz des Blindwertes (ohne Mutarotase) vom Analysenwert (mit Mutarotase) entsprechend folgender Formel:

Enzymaktivität =     $\Delta\Delta$E/min. x 4,09 U Mutarotase pro ml eingesetzter Enzymlösung. (E = Extinktion bei 366 nm)

Rohe Mutarotaseproben sind auf Glucosefreiheit zu prüfen, da der eingebrachte $\beta$-Glucose-Anteil den Test stört.

Alle bislang und im folgenden genannten zur Clonierung eingesetzten Restriktionsenzyme, Plasmid-, Phagen- und Wirtsausgangssysteme sind soweit ihre Herkunft und Herstellbarkeit im Beschreibungstext nicht näher dargelegt ist, bekannt, bzw. in der einschlägigen Literatur ausführlich beschrieben und somit gut zugänglich. Ferner ist eine ausführliche Übersicht hierüber und über die z.T. verwendeten Standardverfahren in (34) wiedergegeben.

Im folgenden sind die Abbildungen erläutert, in denen die einzelnen Teilbereiche der Erfindung in übersichtlicher Form dargestellt sind.

Figur 1:     Reinigung der Mutarotase. Die Reinigung wird durch SDS-Gelelektrophorese nach den einzelnen Reinigungsstufen kontrolliert.
Bahn 1: Rohextrakt;
Bahn 2: Nach Chromatographie an Sephadex G100;
Bahn 3: Nach Chromatographie an CM-Sephadex;
Bahn 4: Nach Chromatographie an Hydroxylapatit;
Bahn S: Molekulargewichts Standard.

Figur 2:     Trennung der Bromcyan-Fragmente durch Gelfiltration über Biogel-B10 und -P30. Mut bedeutet ungespaltenes Material, CB1-5 gibt die Reihenfolge der Bromcyanfragmente innerhalb des Proteins an. CB4 ist mit 11 Aminosäuren (siehe Figur 4) zu klein, um bei der Elution detektiert werden zu können.

Figur 3:     Trennung der tryptischen Peptide durch eine "Reversed phase"-Hochdruckflüssigkeits-Chromatographie.

Figur 3a:     Elutionschromatogramm der ARG C-Fragmente von CB5 auf einer P10-Säule.

Figur 4:     Sequenzierung-Strategie des Mutarotase-Polypeptids und partielle Polypeptid-Sequenz. Die Aminosäuren sind im Einbuchstabencode angegeben. "*" gibt Positionen an, an denen die Aminosäuren nicht bestimmt wurden. Peptide, die durch Spaltung mit Bromcyan, Trypsin oder Endoproteinase ARG C entstanden sind, wurden mit CB, F oder ARG benannt. Die Lokalisation von F42 erfolgte erst nach Sequenzierung des Strukturgens.

Figur 5:     Restriktionskarten der rekombinanten Plasmide pWH1318 und pWH1319. Das rekombinante Plasmid pWH1319 wurde durch Insertion des 1500 bp großen Hind III-Fragments aus pWH1318, das den N-terminalen Teil des Strukturgens enthält, in pBR322 erhalten.

Figur 6a:     Der offene Balken zeigt denselben Bereich wie der offene Balken in Figur 5a (Plasmid pWH1319). Der Pfeil zeigt die 5'-3'-Richtung des Mutarotase-Gens. In diesem Bereich ist das 1500 bp große, mit dem Oligonucleotidgemisch I hybridisierende Hind III-Fragment enthalten.

Figur 6b:     Dargestellt ist das an die proximal zur BclI-Spaltstelle gelegenen Hind III-Spaltstelle anschließende 1500 bp große Hind-III-Fragment, das bei der Analyse der genomischen DNA (Gesamt-DNA-Blot) mit dem Oligonucleotidgemisch II hybridisiert. Der unterstrichene Bereich zeigt  die Überlappung mit dem in Figur 6a abgebildeten DNA-Fragment. Aufgrund dieses überlappenden Bereichs hybridisieren die beiden abgebildeten DNA-Fragmente miteinander.

Figur 7:    Restriktionskarte des M13mp11-Clons pWH1301.

Figur 8:    Kontruktionsschema des rekombinanten Expressionsplasmids pWH1372.

Figur 9:    Aufarbeitung des Polypeptids mit der biologischen Aktivität des Enzyms Mutarotase aus dem Medium. Ein einziger Chromatographieschritt führt zu einer Reinheit, bei der auf einem SDS-Gel keinerlei Verunreinigungen der Mutarotase zu erkennen sind.
Bahn "Mut": Nach $(NH_4)_2SO_4$-Fällung des Mediums;
Bahn "P": Nach Chromatographie an CM-Sephadex.
Bahn "S": Standard-Eichproteine

Figur 10:    DNA-Sequenz des Mutarotase-Gens aus Acinetobacter calcoaceticus (DSM 30008).

Figur 11:    Aminosäure-Sequenz des Expressionsproduktes von pWH1372.

Die folgenden Beispiele erläutern die Erfindung. Dabei werden folgende Abkürzungen verwendet:

| | |
|---|---|
| ATP | Adenosintriphosphat |
| Bis | N,N,N',N'-Methylenbisacrylamid |
| bp | Basenpaare |
| BSA | Rinderserum Albumin |
| CB | Peptide, die durch Bromcyanspaltung entstanden sind |
| cpm | Zählimpulse pro Minute |
| D | Dalton, g/mol |
| DMSO | Dimethylsulfoxid |
| dNTP | Desoxy-Nukleosid-5'-Triphosphat |
| EDTA | Ethylendiamintetraacetat |
| E.coli | Escherichia coli |
| HPLC | Hochdruckflüssigkeitschromatographie |
| IPTG | Isopropylthiogalactosid |
| $^{32}P$ | Phosphorisotop der relativen Masse 32 |
| PEG | Polyethylenglykol |
| PVP | Polyvinylpyrrolidon |
| ARG | Peptide, die durch Spaltung mit Endoproteinase ARG C entstanden sind |
| rpm | Umdrehungen pro Minute |
| SDS | Natriumdodecylsulfat |
| F | Peptide, die durch Spaltung mit Trypsin entstanden sind |
| Tris | Trishydroxymethylaminomethan |
| Trypton | Tryptisch gespaltenes Casein |
| U | Einheit der Enzymaktivität |
| UV | Ultraviolett |
| X-GAL | 5-Bromo-4-Chloro-3-Indolyl-$\beta$-D-Galactopyranosid |
| A,T,C,G | Nucleotide: Adenin, Thymin, Cytosin, Guanin |

Beispiel 1

Isolierung und Reinigung der Mutarotase aus Acinetobacter calcoaceticus

Zunächst wird ein Fermenter mit 100 Liter eines sterilen Nährmediums der folgenden Zusammensetzung beschickt:

| | |
|---|---|
| Pepton aus Casein | 0,5 % |
| Hefeextrakt | 1,0 % |
| Maisstärke-Pulver | 0,3 % |
| Dikaliumhydrogenphosphat | 0,8 % |
| Magnesiumsulfatheptahydrat | 0,04 % |
| Glucose | 1,2 % |
| Silicon-Entschäumer | 10 ml |
| pH-Wert | 6,8 |

Der so vorbereitete Fermenter wird mit 1 Liter einer 18 Stunden inkubierten Submerskultur von

7

Acinetobacter calcoaceticus Stamm 30008, die in der gleichen Nährlösung hergestellt wurde, beimpft.

Diese Kultur wird 18 Stunden bei mittlerer Belüftung und 34 °C inkubiert.

Während der ersten 2 bis 10 Stunden nimmt die Zellmasse, gemessen als Trübung, zu und bleibt dann konstant. Nach 10 Stunden hat die Kultur das Aktivitätsmaximum der Mutarotase mit 43 U/l erreicht.

Die Freisetzung der Mutarotase aus den Bakterien und die Aktivitätsbestimmung erfolgen nach folgenden Verfahren: 3 ml einer gut durchmischten, Glucose-freien Fermenterprobe werden 10 Minuten bei 5000 rpm abzentrifugiert und der Überstand wird verworfen. Die Bakterienmasse wird in 3 ml Phosphat-Puffer 0,1 M, pH 6,5 und 0,1 ml EDTA-Lösung (1,8 g/l) suspendiert, in einem Zentrifugenröhrchen im Aceton-Trockeneis-Kältebad schnell eingefroren und anschließend im 40 °C Wasserbad aufgetaut. Ein Tropfen eines Detergens und 25 bis 30 mg Lysozym werden zugegeben (ca. 15000 U/mg) und bei 28 °C 15 Minuten gerührt. Anschließend wird zur Inaktivierung von NADH-Oxidasen 5 Minuten auf 45 °C erwärmt, aufgeschlossen und das Reaktionsgemisch durch Zentrifugation geklärt. Die Enzymbestimmung erfolgt im so erhaltenen klaren Überstand nach dem vorstehend beschriebenen Verfahren.

Wenn die Kultur das Aktivitätsmaximum erreicht, wird der Mutarotase-Rohextrakt hergestellt. 100 Liter Bakterienschlamm werden mit 0,5 % eines Detergens und 0,1 Mol/l Kaliumchlorid versetzt. Die Zellen werden mit einem Hochdruckhomogenisator bei 500 bar aufgeschlossen. Der Bakterienschlamm muß dabei gekühlt werden. Die Zelltrümmer werden mit einer Zentrifuge 3 Stunden bei 3000 x g abgetrennt. Das Präzipitat wird verworfen und das trübe Zentrifugat wird mit 20 %igem Polyäthylenglykol versetzt, um Begleitsubstanzen auszufällen. Dann wird 30 Minuten gerührt und der Niederschlag abzentrifugiert. Der immer noch trübe Überstand enthält etwa 2 U/ml Mutarotase und wird gegen Wasser diafiltriert bis zu einer Leitfähigkeit von $3 \times 10^{-3}$ S. Nach der Diafiltration wird die Mutarotase bei pH 5,5 im batch-Verfahren an einem trockenen Ionenaustauscher adsorbiert. Für 20.000 U sind 5 bis 10 g Ionenaustauscher-Material erforderlich. Der beladene Ionenaustauscher wird abgesaugt und solange mit 0,025 M Kaliumphosphat-Puffer, pH 5,5, gewaschen, bis das Filtrat klar ist. Danach wird der beladene Ionenaustauscher in eine Säule gepackt, mit 0,025 M Kaliumphosphat-Puffer, pH 5,5, extinktionsfrei gewaschen und anschließend mit einem Kaliumchlorid-Gradienten eluiert.

Die enzymhaltigen Fraktionen werden gesammelt, durch Ultrafiltration konzentriert und anschließend lyophilisiert. Die spezifische Aktivität des so gewonnenen Rohextrakts beträgt etwa 16,1 U/mg Protein.

2420 mg (= 34800 U) des erhaltenen Rohextraktes werden auf eine Molekularsiebsäule Sephadex G100 (20 x 85 cm) aufgetragen. Die Elution erfolgt mit 20 mM Tris-HCl, pH 7,2, die Fließgeschwindigkeit beträgt 70 ml/Stunde, das Auffangvolumen 14 ml/Fraktion. Die Mutarotase-Aktivität wird in den Fraktionen des Eluats gefunden. Die Fraktionen werden so vereinigt, daß ein überlappend eluierendes Protein mit einem Molekulargewicht von 21 KDa ausgeschlossen wird. Dieses läßt sich nämlich nicht durch die nachfolgenden Reinigungsschritte entfernen.

Die vereinigten Fraktionen werden auf eine CM-Sephadex-Säule aufgetragen (2 x 20 cm). Die beladene Säule wird mit 250 ml 20 mM $K_2HPO_4/KH_2PO_4$, pH 9,0, gewaschen. Die Fließgeschwindigkeit beträgt 20 ml/Stunde, das Auffangvolumen 5 ml/Fraktion. Es wird mit einem linearen Gradienten (350 ml 20 mM, 350 ml 170 mM $K_2HPO_4/KH_2PO_4$, pH 9,0) eluiert. Die Elution der Mutarotase erfolgt bei 80 mM Phosphat.

Die zusammengefaßten Proben werden 1:4 verdünnt und auf eine Hydroxylapatitsäule (sphärisch) aufgetragen (4 x 10 cm). Die Fließgeschwindigkeit beträgt 9,2 ml/Stunde, das Auffangvolumen 2,3 ml/Fraktion. Es wird mit 100 ml 20 mM $K_2HPO_4/KH_2PO_4$, pH 9,0, gewaschen. Die Elution erfolgt mit einem linearen Gradienten von 300 ml 20 mM $K_2HPO_4/KH_2PO_4$, pH 9,0, bis 300 ml 300 mM $K_2HPO_4/KH_2PO_4$, pH 9,0, bei dem die Mutarotase bei 85 mM vom Säulenmaterial freigesetzt wird.

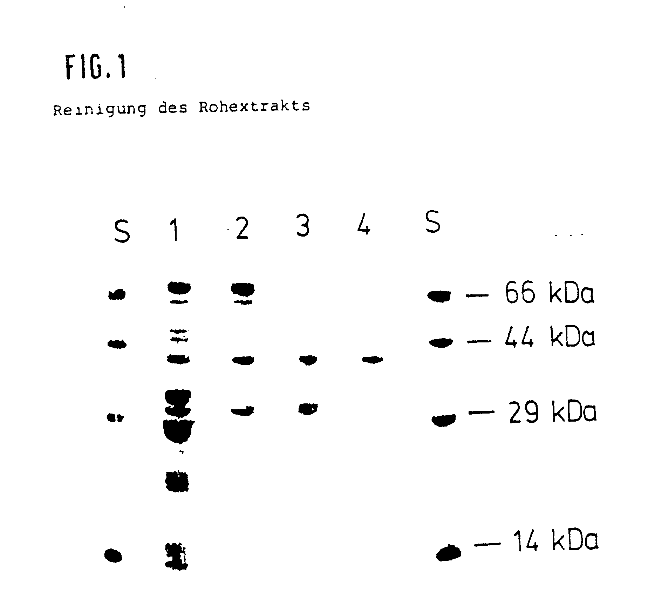

Die Reinigung wird auf einem SDS-Gel (5) verfolgt (Figur 1). Nach dem dritten Säulenschritt ist nur noch eine Bande entsprechend einem Molekulargewicht von 40 KDa auf dem Gel erkennbar. Aus dem Laufverhalten auf der G100-Molekularsiebsäule ist zu entnehmen, daß das Protein unter nativen Bedingungen in oligomerer Form vorliegt.

Tabelle I zeigt eine Zusammenfassung der Reinigungsschritte. Die Proteinbestimmung wird nach Bradford (6) durchgeführt.

Das Trennproblem vereinfacht sich, wenn man statt des ganzen Proteins Bromcyanfragmente in eine enzymatische Verdauung ansetzt. Um weitere Information aus dem C-terminalen Bereich des Proteins zu erhalten, wurde das Bromcyan-Fragment CB5 mit der Endoproteinase ARG C an seinen Arginin-Positionen gespalten.

Die ARG- Spaltung findet ebenfalls in 1M Ammoniumhydrogencarbonat, pH 7-8 in Gegenwart von 1 Gewichtsprozent Proteinase bei 37 °C statt.

Der Verdauungsansatz wurde am Rotationsverdampfer eingeengt, in 2 ml 90 %iger Ameisensäure gelöst, mit 1 ml Wasser verdünnt und auf eine P10-Säule (2,5 x 100 cm) gegeben. Die Elution erfolgte mit 20 %iger Ameisensäure.

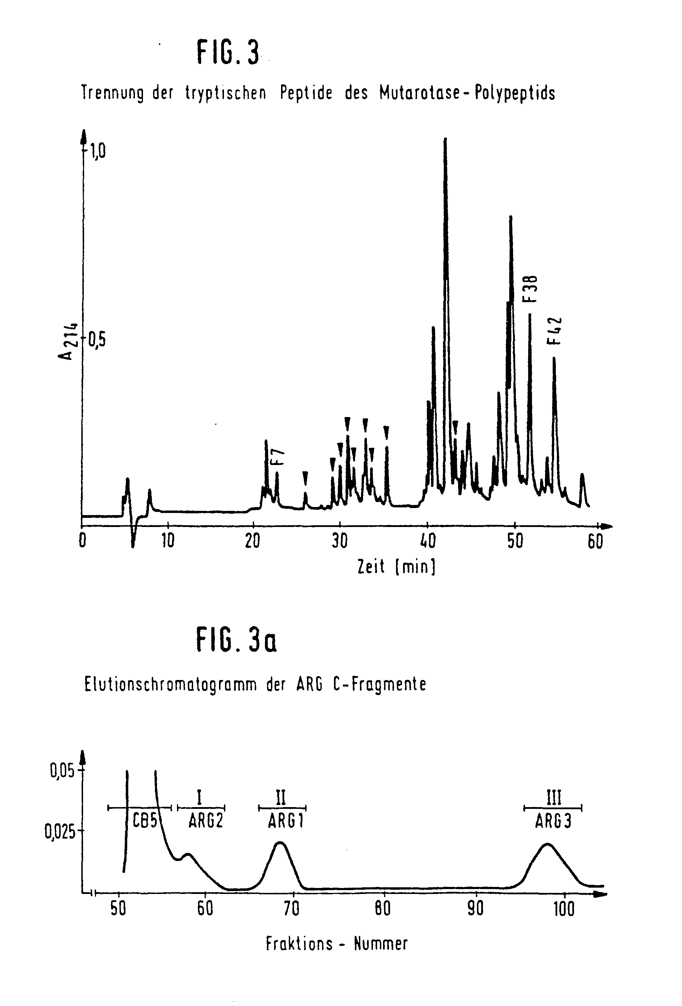

Auf diese Weise ließen sich die drei Fraktionen I bis III eluieren, von denen Fraktion I noch einmal über eine 2,5 x 50 cm lange P10-Säule rechromatographiert wurde (Fig. 3a).

Aminosäure-Analysen

Zunächst wird eine Aminosäure-Analyse des Mutarotase-Gesamtpolypeptids durchgeführt. Dazu werden 5 nMol Protein unter reduzierenden Bedingungen in 2 ml 6 M HCl, 0,05 % Thioglykol, 2 Stunden bei 150 °C hydrolysiert Die Hydrolyse erfolgt 20 Stunden unter oxidativen Bedingungen in 2 ml 6 M HCl, 1,5 % DMSO bei 110 °C. Dann wird die Probe am Rotationsverdampfer eingeengt und nach den Angaben des Herstellers mit dem Aminosäure-Analysator LC 600 (Biotronik, Frankfurt) auf einer 0,6 x 21 cm DC 6A Harz-Säule analysiert.

Die so ermittelte Aminosäurezusammensetzung des Gesamtpolypeptids wird in Tabelle II gezeigt. Die aus der DNA-Sequenz abgeleitete Aminosäurezusammensetzung ist in der Spalte Sequenz aufgelistet. Daraus ergibt sich ein berechenbares Molekulargewicht der Mutarotase von 39500 Da.

Tabelle I

| Reinigungsschema der Mutarotase | | | |
|---|---|---|---|
| | Aktivität (U) | Spez./Aktivität (U/mg) | Ausbeute in % |
| Rohextrakt | 34800 | 16.5 | 100 |
| Hydroxylapatit | 27885 | 68.0 | 80 |
| CM-Sephadex | 19841 | 107.0 | 57 |
| Hydroxylapatit | 15656 | 233.0 | 45 |

Beispiel 2

Partielle Bestimmung der Peptidsequenz der Mutarotase

Mit Hilfe der im folgenden beschriebenen Methoden wird eine Teilsequenz des Mutarotase-Peptids ermittelt werden, in der Aminosäuren enthalten sind, die von möglichst wenig verschiedenen Codons auf der DNA codiert werden. Solche Sequenzen sind später für die Auswahl zugehöriger Oligonucleotide (vgl. Beispiel 3) besonders gut geeignet.

Das nach dem letzten säulenchromatographischen Reinigungsschritt gemäß Beispiel 1 in Phosphatpuffer vorliegende Protein wird mit einem halben Volumen 100prozentiger Essigsäure und mit einem halben Volumen n-Propanol versetzt und am Rotationsverdampfer zur Trockne eingeengt. Zur Entsalzung wird es auf eine P 10 Molekularsiebsäule (2,5 x 25 cm) gegeben und mit 70prozentiger Essigsäure eluiert. Der Nachweis des Proteins in den einzelnen Fraktionen erfolgt durch Messung der Absorption bei 280 nm. Protein-enthaltende Fraktionen werden vereinigt und am Rotationsverdampfer zur Trockne eingeengt. Das auf diese Weise erhaltene Protein (Mutarotase) wurde für die anschließende Sequenzierung der N-terminalen Aminosäuren sowie für die Bromcyan- und Trypsin-Spaltungen verwendet.

Bromcyan-, Trypsin- und Endoproteinase ARG C-Spaltungen

Die Spaltungen mit Bromcyan und mit den Enzymen Trypsin und Endoproteinase ARG C werden zur Bestimmung von Teilaminosäuresequenzen der Mutarotase durchgeführt.

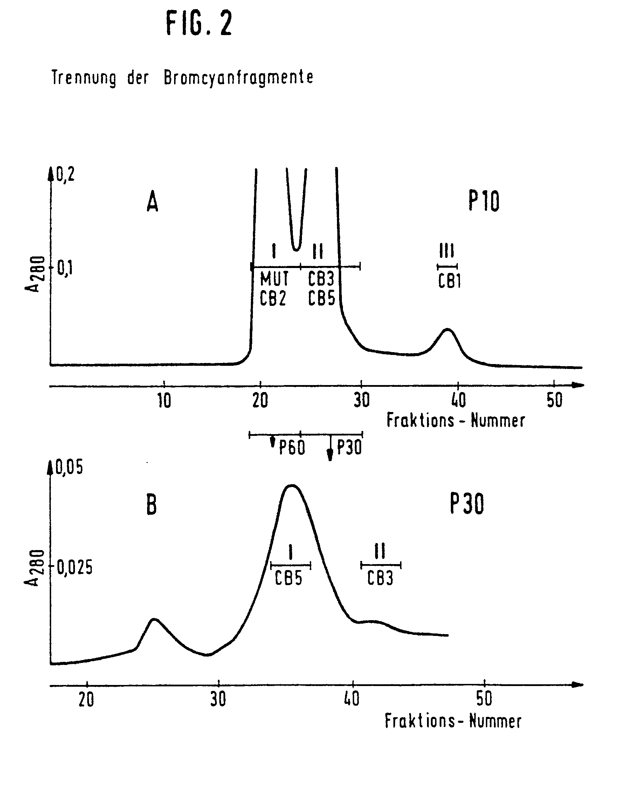

Die Spaltung mit Bromcyan erfolgt nach der Vorschrift von Gross und Wittkopp (12). 15 mg des wie vorstehend beschrieben entsalzten und eingeengten Proteins werden in 3 ml 70prozentiger Ameisensäure aufgenommen, mit einem 100fachen molaren Überschuß Bromcyan versetzt und 7 Stunden im Dunkeln inkubiert. Die Ameisensäure wird im Rotationsverdampfer entfernt und der Rückstand wird in 2 ml 70prozentiger Essigsäure aufgenommen. Dann werden die Spaltprodukte auf einer P 10- und einer P 30-Molekularsiebsäule (2,5 x 50 cm) getrennt. Die einzelnen Spaltprodukte (Fragmente) werden als CB1-5 bezeichnet. Figur 2 zeigt, mit welcher Fraktion die einzelnen Fragmente bei der beschriebenen Säulenchromatographie eluiert werden.

Für die Verdauung des Gesamtproteins mit Trypsin werden 2 mg entsalztes und zur Trockne eingeengtes Protein in 2 ml 1 M Ammoniumhydrogencarbonat gelöst und mit einem Gewichtsprozent Proteinase versetzt. Da sich das Gesamtprotein unter diesen Bedingungen nicht löst, wird die Mischung auf 0,05 % SDS eingestellt. Insgesamt muß 4 x Trypsin zugegeben werden. Nach der letzten Zugabe wird über Nacht inkubiert. Das Reaktionsgemisch, das immer noch unlösliche Anteile enthält, wird in Aliquots a 100 µl auf eine HPLC Säule gegeben.

Die HPLC-Anlage von Beckman besteht aus zwei Lösungsmittel-Spendern (Modell 100 A und 110 A), einem variablen Wellenlängendetektor (Modell 165) und einem Gradientenmischer. Die Elution beginnt mit Lösung A (0,05 % Trifluoressigsäure in Wasser), 10 Minuten. Danach wird ein Gradient angelegt, bei dem die Konzentration des Lösungsmittels B (0,025 % Trifluoressigsäure in Acetonitril) innerhalb von 50 Minuten auf 50 % ansteigt. Die Durchflußrate beträgt 1,6 ml/min, die Temperatur 50 °C. Die Säule (0,72 x 25 cm) ist mit einem kugelförmigen Kieselgel (Nukleosil 5 $C_{18}$, Machery & Nagel, Düren) gefüllt. Die Peptide werden durch Messungen der Absorption bei 216 nm identifiziert. Für die Sequenzierung werden die Fraktionen von 7 bis 10 Chromatographieläufen gesammelt.

Auf diese Weise werden unter anderem die Trypsin-Fragmente F38 und F42 des Mutarotase-Gesamtpolypeptids isoliert (vgl. Figur 3).

Die tryptische Verdauung des Gesamtproteins führt z.T. zu schwer auftrennbaren Spaltprodukten.

## Tabelle II

| Aminosäure | Oxid.Bed. | Red.Bed. | Sequenz | |
|---|---|---|---|---|
| ASX | 48,56 | 51,71 | 53 | ASN 33 |
| | | | | ASP 20 |
| THR | 23,13 | 22,45 | 27 | |
| SER | 19,89 | 18,25 | 23 | |
| GLX | 38,62 | 38,12 | 33 | GLN 25 |
| | | | | GLU 8 |
| PRO | 19,57 | 18,78 | 18 | |
| GLY | 36,32 | 38,46 | 33 | |
| ALA | 27,00 | 24,38 | 20 | |
| CYS | - | - | - | |
| VAL | 23,34 | 27,82 | 31 | |
| MET | 4,70 | 4,04 | 4 | |
| ILE | 13,50 | 15,52 | 15 | |
| LEU | 29,30 | 29,88 | 30 | |
| TYR | 13,08 | 13,71 | 15 | |
| PHE | 14,65 | 15,25 | 15 | |
| HIS | 12,66 | 7,01 | 6 | |
| LYS | 23,23 | 24,23 | 25 | |
| ARG | 11,20 | 9,30 | 9 | |
| TRP | ? | 2 | 2 | |
| | 359 | 359 | 361 | |

Tryptophan kann unter den genannten Versuchsbedingungen nicht ermittelt werden.

Aus Tabelle II ist zu entnehmen, daß das Protein vier Methionin-Reste enthält. Daher werden bei der Bromcyan-Spaltung fünf Fragmente erwartet. Somit stimmen die Ergebnisse aus der vorstehend beschriebenen Bromcyan-Spaltung und der anschließenden Trennung der Spaltprodukte (vgl. Figur 2) mit den Ergebnissen der Aminosäure-Analyse (vgl. Tabelle II) überein.

Gegebenenfalls wird auch die Aminosäurezusammensetzung der durch die Bromcyan-, Trypsin- und Endoproteinase ARG C-Spaltung erhaltene Peptide wie vorstehend beschrieben ermittelt.

Bestimmung von Aminosäuresequenzen

Zunächst werden die N-terminalen Aminosäuren der Mutarotase mit einem automatischen Flüssigphasen-Sequenzierungsgerät (Beckman Modell 890C) bestimmt. Die 2-Anilino-1,3-thiazolin-5-on-Derivate der Aminosäuren werden bei 55 °C in 20 % Trifluoressigsäure 30 Minuten inkubiert. Die Phenylthiohydantoin-Derivate werden auf einer HPLC-Anlage identifiziert (8).

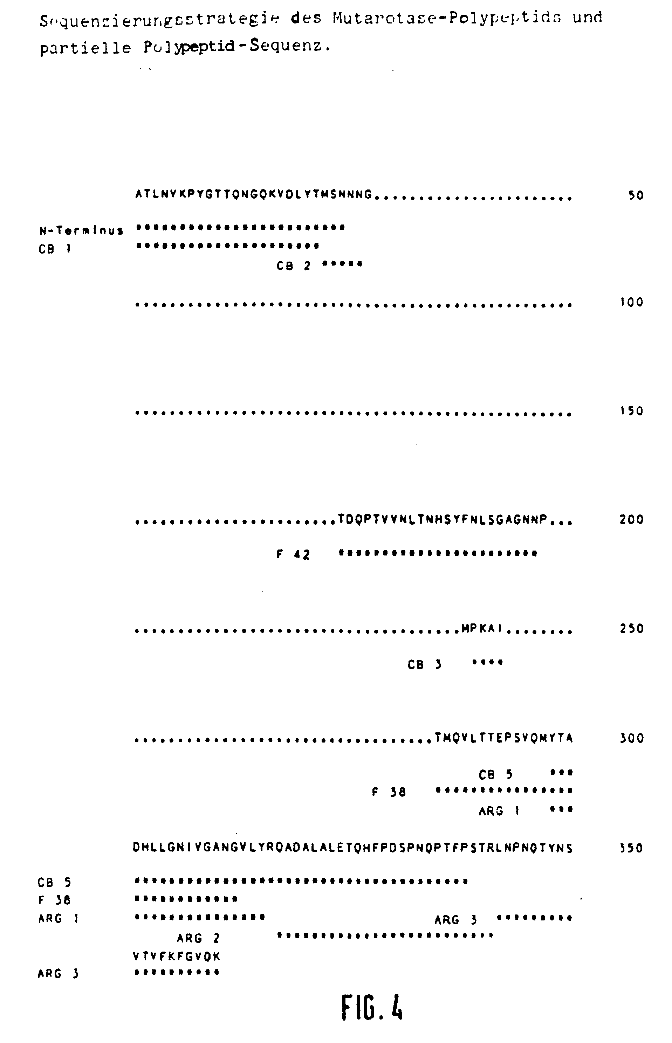

Nachfolgend werden Aminosäuresequenzen der Fragmente CB1, CB2, F42, CB3, CB4, CB5, F38, ARG1, ARG2 und ARG3 bestimmt (vgl. Figur 4).

Die Sequenzierung dieser Fragmente erfolgt bei 50 °C nach der Festphasentechnik unter Verwendung eines LKB-Geräts, Modell 4020. Die Fragmente werden C-terminal mit EDC (1-Äthyl-3-(3'-diäthylaminopropyl)-carbodiimid-HCl)an 3-Aminopropylglas gekoppelt (9). Der anschliessende Edman-Abbau wird nach einer Vorschrift von Laurson (10) ausgeführt. Die Phenylhydantoin-Derivate der Aminosäuren werden dünnschichtchromatographisch auf Merck HPTLC-Platten (Kieselgel 60F G 254) identifiziert (11).

Die so erhaltenen Aminosäuresequenzen der genannten Mutarotase-Fragmente werden anschließend ihren relativen Positionen in der Gesamtaminosäure-Sequenz der Mutarotase zugeordnet.

Dies wird wie folgt durchgeführt:

Die Lage der Bromcyan-Fragmente CB1 und CB2 ergibt sich aus der Übereinstimmung ihrer Aminosäure-sequenz mit N-terminalen Aminosäure-Sequenzen des Gesamtpolypeptids (vgl. Figur 4).

Vom Bromcyan-Fragment CB3 werden die ersten fünf Aminosäuren bestimmt. Das absorbierende Material des ersten Maximums bei der auf die Bromcyanspaltung folgenden Chromatographie wird auf einer P60-Säule (2,5 x 50 cm) rechromatographiert. Die Sequenzierung zeigt parallel die N-terminale Sequenz (d.h. das Maximum enthielt ungespaltenes Material) sowie die ersten sechs Aminosäuren des Bromcyan-Fragmentes CB2. Die relative Lage von CB3 im Mutarotase-Polypeptid kann erst nach der Bestimmung der DNA-Sequenz des entsprechenden Strukturgens ermittelt werden (vgl. Figur 4).

Die Sequenz des Bromcyan-Fragments CB4 ist in dem tryptischen Fragment F 38 vollständig enthalten. Es umfaßt elf Aminosäuren (vgl. Figur 4).

Das Bromcyan-Fragment CB5 kann durch eine Rechromatographie über eine 2,5 x 50 cm große P30-Säule vom Bromcyan-Fragment CB3 abgetrennt werden. Da in der Aminosäure-Analyse kein Homoserin zu finden ist, muß es sich beim Bromcyan-Fragment CB5 um das C-terminale Fragment handeln. Von CB5 werden 42 Aminosäuren sequenziert (vgl. Figur 4).

Die relative Zuordnung der ermittelten Sequenzen der Bromcyan-Fragmente CB4 und CB5 zur Gesamtpolypeptid-Sequenz der Mutarotase wird über die Sequenzierung von tryptischen Fragmenten des Mutarotase-Polypeptids und des Bromcyan-Fragments CB5 des Mutarotase-Polypeptids ermittelt.

Die aus der Subfragmentierung des Bromcyan-Fragmentes CB5 resultierenden Fraktionen I, II und III (Fig. 3a) wurden sequenziert. Die Sequenz des Peptids in Fraktion II ist mit den ersten 20 Aminosäuren von CB5 identisch.

Die Peptide wurden von ARG1 bis ARG3 nach ihrer Reihenfolge innerhalb des Bromcyanfragmentes numeriert. Die Sequenz des Peptids in Fraktion II (ARG1) war mit den ersten 20 Aminosäuren von CB5 identisch. Das Peptid aus Fraktion 1 konnte als ARG2 identifiziert werden, da seine Sequenz mit den Positionen 21 bis 42 von CB5 überlappte. Es ging über diese Sequenz um die Aminosäuren Ser-Thr-Arg hinaus. Bei ARG3 handelt es sich um die C-terminale Sequenz von CB5. Die Sequenz von ARG1, ARG2 und ARG3 sind in Fig. 4 aufgeführt.

Weil das Bromcyan-Fragment CB5 das C-terminale Peptid-Fragment der Mutarotase ist, steht somit die C-terminale Aminosäure-Sequenz des Mutarotase-Polypeptids fest (vgl. Figur 4).

Die Sequenzierung von 25 Aminosäuren des tryptischen Fragments F38 des Gesamtproteins ergibt die relative Lage der Bromcyan-Fragmente CB4 und CB5 zueinander. Da F38 mit CB4 und CB5 überlappt, müssen CB4 und CB5 direkt benachbart sein (vgl. Figur 4).

Schließlich wird die Aminosäuresequenz des vom Mutarotase-Gesamtpolypeptid abgeleiteten tryptischen Fragments F42 ermittelt. Die relative Position der 25 Aminosäuren langen Sequenz kann erst nach der Bestimmung einer Nucleotid-Teilsequenz des Mutarotase-Strukturgens festgelegt werden (vgl. Tabelle V).

Beispiel 3

Synthese von Oligonucleotiden

Das für die Mutarotase codierende Gen wird gemäß Beispiel 5 aus einer Genbank isoliert.

Als Sondenmoleküle für das Absuchen dieser Genbank werden Oligodesoxynucleotide (kurz: Oligonucleotide) durch chemische Synthese hergestellt.

Die Sequenz dieser Oligonucleotide wird von den Aminosäuren 12 bis 17 und 353 bis 359 (den letzten 7 Aminosäuren) der gemäß Beispiel 2 ermittelten partiellen Aminosäure-Sequenz des Mutarotase-Polypeptids abgeleitet.

Da der genetische Code degeneriert ist, müssen verschiedene Oligonucleotide synthetisiert werden, die dieselbe Aminosäure-Sequenz codieren. Die jeweils in Frage kommenden Oligonucleotide sind in Tabelle III aufgeführt.

## Tabelle III

### Oligonucleotidgemisch I

| Position im Muta-rotase-Polypeptid | 12 | 13 | 14 | 15 | 16 | 17 |
|---|---|---|---|---|---|---|
| Dreibuchstabencode | GLN | ASN | GLY | GLN | LYS | VAL |
| Einbuchstabencode | Q | N | G | Q | K | V |
| Sequenz der Oligonucleotide | $CA_{A}^{G}$ | $AA_{T}^{C}$ | GGT | $CA_{A}^{G}$ | $AA_{A}^{G}$ | GG |

### Oligonucleotidgemisch II

| Position im Muta-rotase-Polypeptid | 353 | 354 | 355 | 356 | 357 | 358 | 359 |
|---|---|---|---|---|---|---|---|
| Dreibuchstabencode | PHE | LYS | PHE | GLY | VAL | GLN | LYS |
| Einbuchstabencode | F | K | F | G | V | Q | K |
| Sequenz der Oligonucleotide | $TT_{C}^{T}$ | $AA_{A}^{G}$ | $TT_{C}^{T}$ | GGT | GTT | $CA_{A}^{G}$ | $AA_{A}^{G}$ |

Der Dreibuchstabencode und der Einbuchstabencode sind die üblichen Abkürzungen für die in der Natur vorkommenden 20 biogenen Aminosäuren.

Alle aus der Tabelle III zu entnehmenden Kombinationen der Sequenzen der Oligonucleotide werden gleichzeitig synthetisiert. Die Synthese wird an einem mit funktionellen n-Propylaminogruppen versehenen Silicagel als Träger nach der von Caruthers et al. entwickelten Phosphoramidit-Methode durchgeführt (14, 15). Wenn an der dritten Position der einzelnen Triplets zwei Nucleotide möglich sind, die miteinander keine Wasserstoffbrückenbindung bilden können, werden äquimolare Mengen der Phosphoramidite für die Kopplung angeboten. Wenn G und C sowie A und T alternativ eingesetzt werden, wird das Trägermaterial für diesen einen Kopplungsschritt geteilt. Am Ende der Synthese wird der Detritylierungsschritt nicht durchgeführt, da das gewünschte Oligonucleotid durch die Tritylschutzgruppe am 5'-Ende deutlicher von Nebenprodukten zu unterscheiden ist. Nach Abspaltung der Phosphatschutzgruppen wird die kovalente Bindung zum Trägermaterial hydrolysiert und die Basenschutzgruppen abgespalten. Die Lösung wird dann im Rotationsverdampfer zur Trockne eingeengt und in einem Puffer aufgenommen (10 mM Tris-HCl, pH 8,0, 1 mM EDTA). Schwebestoffe und Trägerreste werden aus dem so erhaltenen Rohgemisch von Oligonucleotiden durch Zentrifugation abgetrennt. 5 bis 15 $A_{260}$-Einheiten werden jeweils in einem Lauf über eine HPLC-Säule gereinigt. Die Elution bei dieser Säulenchromatographie beginnt mit Lösung A (20 % Acetonitril in 0,1 M Triäthylammoniumacetat), 5 Minuten. Danach wird ein Gradient angelegt, bei dem die Acetonitrilkonzentration innerhalb von 30 Minuten auf 30 % ansteigt. Die Durchflußrate beträgt 15 ml/min, die Temperatur 45 °C. Die Oligonucleotide enthaltenden Eluate werden vereinigt und zur Trockne eingengt, mit 400 µl 80prozentiger (v/v) Essigsäure versetzt und nach 5 Minuten bei Raumtemperatur zur Trockne evaporiert. Der Rückstand wird in 500 µl Wasser resuspendiert und mit einem Gradienten von 5 % bis 20 % Acetonitril rechromatographiert. Die detritylierten Oligonucleotide werden bei dieser Chromatographie zwischen einer 13- und 17prozentigen Acetonitrilkonzentration eluiert.

Schließlich wird die Endausbeute bei 260 nm mit HPLC-Puffer als Vergleichslösung bestimmt und die beiden erhaltenen Oligonucleotidgemische I und II werden zweimal aus Wasser gefriergetrocknet und in 10 mM Tris-HCl, pH 8,0, 0,1 mM EDTA-Puffer suspendiert.

Beispiel 4

Präparation chromosomaler DNA aus Acinetobacter calcoaceticus

Gemäß Beispiel 1 werden Zellen von Acinetobacter calcoaceticus (DSM 30008) gezüchtet.

5 g Zellen wurden in 50 mM NaCl, 50 mM EDTA, 30 mM Tris-HCl, pH 7,9, suspendiert und mit 200 mg Lysozym 30 Minuten bei 37 °C inkubiert. Die Suspension wird auf eine Konzentration von 1 % SDS gebracht und mit 5 mg Proteinase K (Merck) weitere 30 Minuten bei 37 °C inkubiert. Danach wird vorsichtig sechsmal mit Phenol (äquilibriert mit 10 mM Tris-HCl, pH 8,0, 0,1 mM EDTA) und anschließend viermal mit Chloroform/Isoamylalkohol (20:1) extrahiert.

Dann wird die enthaltene DNA durch Zugabe eines dreifachen Volumens Äthanol gefällt. Die DNA wird abzentrifugiert, der Niederschlag wird getrocknet und in 5 ml 10 mM Tris-HCl, pH 8,0, 0,1 mM EDTA resuspendiert. Anschliessend werden 50 µg DNAse-freie RNAse A(Sigma) zugegeben. Die DNAse ist vorher durch 10minütiges Kochen in 100 mM NaOAc, pH 5,5, inaktiviert worden. Nach 30minütiger Inkubation mit RNAse A werden 250 µg Proteinase K zugegeben und es wird nochmals 30 Minuten inkubiert. Danach wird wieder sechsmal mit Phenol und dreimal mit Diäthyläther ausgeschüttelt. Die DNA wird dann durch gabe des halben Volumens einer 30prozentigen PEG-Lösung in 1,5 M NaCl ausgefällt, mit Äthanol gespült und im Exsikkator getrocknet.

Beispiel 5

Clonierung genomischer Mutarotase-Sequenzen

Material und Methoden

Enzyme: Restriktionsenzyme, T4-DNA-Ligase und E.coli-Polymerase sowie das Klenow-Fragment der E.coli-Polymerase wurden von Boehringer, BRL oder Biolabs gekauft und nach den Angaben des Herstellers verwendet. Eco RI wird nach einer Vorschrift von Greene et al. (16) präpariert.

Gel-Elektrophoresen: Für Restriktionsanalysen werden DNA-Fragmente bis zu einer Länge von 1000 bp auf 5 % Polyacrylamidgelen (Acrylamid/bis-Acrylamid 20:1) in TEB-Puffer (60 mM Tris, 60 mM Borsäure, 1 mM EDTA, pH 8,3) und 10 % Glycerin aufgetrennt (17). Zur Auftrennung von DNA-Fragmenten einer Länge von 500 bp bis 9000 bp werden 1 % Agarose-Gele in TAE-Puffer (40 mM Tris, 15 mM NaOAc, 1,25 mM EDTA, pH 8,3) verwendet (18). Bei vertikalen Agarosegel-Elektrophoresen wird in den unteren Teil der Apparatur ein 3 cm hoher 8 % Polyacrylamid-Stützblock gegossen. Da sich aus Agarose-Gelen isolierte DNA nicht für Clonierungsversuche eignet, wird für präparative Zwecke ein 3 % Polyacrylamidgel (Acrylamid/bis-Acrylamid 50:1) ohne Zusatz von Glycerin verwendet. Nach der Gel-Elektrophorese werden die Gele aus der Apparatur ausgebaut und 10 Minuten in einer 0,001 prozentigen Ethidiumbromidlösung angefärbt. Dadurch werden die DNA-enthaltenen Banden im UV-Licht sichtbar.

Präparation von DNA: Die Präparation chromosomaler DNA aus Acinetobacter calcoaceticus ist in Beispiel 4 beschrieben.

Die Isolation von Plasmid DNA und von DNA der RF-Form des M13-Phagen erfolgt nach der Methode von Hardies et al. (19). Die Präparation einzelsträngiger M13-DNA wird wie von Messing (20) beschrieben durchgeführt. Für gelelektrophoretische Auftrennungen oder Hybridisierungsanalysen werden die Phagenüberstände durch Fällung mit 1/4 Volumen 20prozentiger PEG 6000-Lösung in 2,5 M NaCl 15 Minuten bei 4 °C fünffach konzentriert.

Die präparative Isolierung von Plasmid-DNA wird gemäß der Vorschrift zur Schnellanalyse von Transformanten wie folgt ausgeführt.

Einzelne Bakterienkolonien werden auf einer Selektionsplatte ausplattiert. Mit einer Impföse wird etwa 1 mm³ Bakterienmasse entnommen und in 80 µl Tritonpuffer (8 % Saccharose, 5 % Triton X100, 50 mM EDTA, 50 mM Tris-HCl, pH 8,0) suspendiert. Nach Zugabe von 7 µl Lysozympuffer (10 mg/ml Lysozym in 50 mM Tris-HCl, pH 8,0, 50 mM EDTA) werden die Proben 5 Minuten bei Raumtemperatur inkubiert. Dann werden sie 40 Sekunden auf 100 °C erhitzt und anschließend 2 Minuten auf Eis gestellt. Nach 15minütiger Zentrifugation in einer Mikrozentrifuge werden die schleimigen Überreste der Bakterien mit einer Impföse entfernt. Der Überstand wird dann mit 80 µl Isopropanol versetzt und 20 Minuten in ein Eisbad gestellt. Der dabei entstehende Niederschlag wird durch 15minütiges Zentrifugieren sedimentiert, nach Verwerfen des Überstandes zweimal mit 600 µl Äthanol gewaschen und im Vakuum getrocknet. Die erhaltene Plasmid-DNA wird in 40 µl TE-Puffer (10 mM Tris-HCl, pH 8,0, 0,1 mM EDTA) gelöst und auf einem Agarose-Gel analysiert.

Gelelutionen:

Die Elution von DNA-Fragmenten aus Polyacrylamidgelen wird nach der Methode von Maxam und Gilbert (21) durchgeführt. 5prozentige und 20prozentige Polyacrylamidgelstücke werden zunächst mit einem Teflonstab mechanisch zerkleinert. 3prozentige Polyacrylamidgelstücke werden mit einer 20 ml Einmalspritze durch eine Kanüle mit einem Durchmesser von 0,8 mm gepreßt.

Die eluierte DNA wird auf eine DE-52-Säule mit einem Säulenmaterialvolumen von 200 $\mu$l aufgetragen und mit 400 $\mu$l 2 M NaCl eluiert (20). Oligonucleotide werden mit 1 M NaCl eluiert und dann direkt zur Hybridisierung verwendet. Doppelsträngige DNA wird nach der Elution von der DE-52-Säule noch zweimal mit Äthanol gefällt.

Transformation:

Die Transformation von E.coli-Stämmen erfolgt nach der CaCl$_2$-Methode (17).

Radioaktive Markierung von DNA:

Oligonucleotide werden an ihrem 5'-Ende mit Hilfe der T4-Polynucleotidkinase unter Verwendung von [$\gamma$-$^{32}$p]-ATP radioaktiv markiert (21). Das dazu benötigte [$\gamma$-$^{32}$p]-ATP mit einer spezifischen Aktivität von 8000 Ci/mMol wird nach der Methode von Walseth und Johnson (23) synthetisiert. Die spezifische Aktivität der erhaltenen markierten Oligonucleotide wird durch die Abtrennung nicht phosphorylierter Oligonucleotide auf einem 40 cm langen 20prozentigen 8M Harnstoffgel erhöht. Durch diese Gel-Elektrophorese wird gleichzeitig freies ATP und anorganisches Phosphat abgetrennt.

Plasmid-DNA wird nach der "Nick-Translations"-Methode von Weinstock (24) radioaktiv markiert. Im Reaktionsgemisch verbleibende überschüssige Triphosphate werden anschließend über eine G50-Molekularsiebsäule in einer Pasteur-Pipette abgetrennt.

Hybridisierung:

Southern-Blot-Analyse:

Die durch Spaltung chromosomaler DNA oder von DNA rekombinanter Plasmide erhaltenen Restriktionsfragmente werden auf Agarose-Gelen aufgetrennt und nach Southern (25) auf Nitrocellulose geblottet. Der Nitrocellulose-Filter wird in Haushaltsfolie eingeschweißt und mit 1 ml Hybridisierungslösung pro 40 cm$^2$ Filterfläche 4 Stunden bei 60 °C vorhybridisiert (6 x NET, 10 x Denhardt-Lösung, 0,1 % SDS; 1 x NET = 0,15 M NaCl, 15 mM Tris-HCl, pH 8,3, 1 mM EDTA; 1 x Denhardt-Lösung = 0,02 % Rinderserumalbumin, 0,02 % Polyvinylpyrrolidon-40, 0,02 % Ficoll). Danach wird das markierte Oligonucleotid (ca. 1-2 x 10$^6$ cpm bei einer Cerenkov-Zählung) zugegeben. Bei beiden Oligonucleotidgemischen wird als Hybridisierungstemperatur 40 °C gewählt. Nach 3 bis 5stündiger Hybridisierung werden die Filter zweimal 15 Minuten bei 4 °C gewaschen. Danach werden sie 1 Minute bei 40 °C gewaschen. Die anschließende Autoradiographie wird dann 12 Stunden bei -70 °C durchgeführt. Unspezifische Hybridisierungs-Signale können durch einen weiteren 1minütigen Waschvorgang bei höherer Temperatur beseitigt werden. Die entsprechenden Nitrocellulosefilter werden dann wie vorstehend beschrieben nochmals belichtet.

Dot-Blot-Analyse:

Die wie vorstehend beschrieben präparierte DNA rekombinanter Plasmide, aus 3prozentigen Polyacrylamidgelen eluierte chromosomale DNA und einzelsträngige Phagenüberstände werden einer Dot-Blot-Analyse unterzogen. Dazu werden 5 $\mu$l DNA auf einen Nitrocellulosefilter pipettiert und mit einem Föhn angetrocknet. Bei doppelsträngiger DNA wird der Filter auf angefeuchtetem Chromatographiepapier bei Raumtemperatur weiterbehandelt. Es werden nacheinander folgende Lösungen, mit denen das Chromatographiepapier getränkt war, verwendet:

5 Minuten 250 mM Tris-HCl, pH 7,5; 5 Minuten 500 mM NaOH; 5 Minuten 500 mM NaOH/1,5 M NaCl; 2 x 2 Minuten 1 M Tris-HCl, pH 7,5; 4 Minuten 500 mM Tris-HCl, pH 7,5/1,5 M NaCl; 5 Minuten 6 x SSC-Puffer (1 x SSC = 150 mM NaCl, 15 mM tri-Natriumcitrat). Anschließend wird der Filter 2 Stunden bei 80 °C in einem Ofen unter vermindertem Druck gebacken. Die Hybridisierung der Filter mit den radioaktiv markierten Oligonucleotidgemischen I und II erfolgt wie vorstehend beschrieben. Die Hybridisierung der Filter mit "Nick-translatierten" Plasmiden wird nach der Vorschrift von Wahl et al. (26) durchgeführt.

Koloniehybridisierung:

Je eine von Zwei Agarplatten (angereichert mit 40 mg/Liter Ampicillin) (24,3 x 24,3 cm, Nunc Intermed, Screening Plate) wird mit einem Nitrocellulose-Filter (Schleicher und Schüll BA-85) abgedeckt. Jeweils 1000 Bakterienkolonien werden dann mit sterilen Zahnstochern im gleichen Muster auf den Nitrocellulose-Filter der einen Platte und den Agar der anderen Platte übertragen und 18 Stunden bei 37 °C inkubiert. Die Filter mit den Kolonien werden im folgenden analog zur Dot-Blot-Analysen-Vorschrift (s. oben) behandelt. Nach dem 2stündigen Backen bei 80 °C unter vermindertem Druck werden die Filter dreimal mit 6 x NET, 10 x Denhardt-Lösung, 0,1 % SDS gewaschen und im selben Puffer mit 24 x 10$^6$ cpm markierten Oligonucleoti- den 5 Stunden bei 40 °C hybridisiert. Anschließend werden die Filter mit 6 x SSC zweimal 15 Minuten bei 4 °C gewaschen, dann 5 bis 8 Minuten bei 40 °C und schließlich 1 Minute bei 42 °C. Nach der ersten Belichtung eines Röntgenfilms werden die Filter nochmals 2 Minuten bei 44 °C mit 6 x SSC gewaschen und zur Belichtung eines weiteren Röntgenfilms verwendet. Schließlich werden die Filter 1 Minute bei 46 °C in 6 x SSC gewaschen und nochmals zur Belichtung eines Röntgenfilms eingesetzt.

Sequenzanalysen:

Die Bestimmung von DNA-Sequenzen wird größtenteils nach Maxam und Gilbert durchgeführt (21). Gegebenenfalls, z.B. wenn keine geeigneten Restriktionsspaltstellen vorhanden waren, werden die DNA- Fragmente in pUR250 subcloniert (28). Zur Identifizierung von M13-Clonen wird eine Sequenzierung nach der M13-Methode (27) durchgeführt.

Die genannten Methoden sind im wesentlichen molekularbiologische Standardverfahren, die in Laborhandbüchern, wie "Molecular Cloning, A Laboratory Manual", von T. Maniatis et al., Cold Spring Harbor Laboratory, 1982, beschrieben sind.

Clonierung genomischer Mutarotase-Sequenzen:

Je 400 μg chromosomale DNA aus Acinetobacter calcoaceticus (DSM 30008) werden mit den Restriktionsendonucleasen Eco RI, Hind III und Bcl I gespalten. Dann werden 20 μg auf einem 1prozentigen Agarose-Gel elektrophore siert und auf Nitrocellulose geblottet (sogenannter Gesamt-DNA-Blot). Nach der wie vorstehend beschrieben durchgeführten Hybridisierung wird beim Oligonucleotidgemisch I ein zweites Mal bei 44 °C nachgewaschen, beim Oligonucleotidgemisch II ein zweites Mal bei 46 °C.

Die Hybridisierung der Bcl I-Spaltung mit dem Oligonucleotidgemisch I ergibt reproduzierbare Signale im Größenbereich von 6400 bp. Bei der Hybridisierung der Eco RI-Spaltung mit dem Oligonucleotidgemisch I ergeben sich reproduzierbare Signale im Größenbereich von 2000 bp und bei der Hind III-Spaltung im Größenbereich von 1500 bp. Mit dem Oligonucleotidgemisch II hybridisiert ein ca. 1500 bp großes Hind III- Fragment.

Anschließend wird mit den Bcl I-Fragment der Acinetobacter calcoaceticus-DNA und Bam HI-gespaltener DNA des Plasmids pBR327 (ATCC 31344) eine Genbank konstruiert.

Dazu werden 120 μg BclI-gespaltene chromosomale DNA von Acinetobacter calcoaceticus auf einem 3prozentigen Polyacrylamid-Gel elektrophoretisiert, bis Fragmente einer Länge von 3800 bp die untere Kante des Gels erreicht haben. Das Acrylamid oberhalb 3800 bp wird in 9 schmale Streifen geschnitten und die DNA aus jeder einzelnen Gelfraktion eluiert. Je 1/4 der eluierten DNA wird in einer Dot-Blot-Analyse hybridisiert. Die Fraktion aus dem Gelstreifen mit den zweitgrößten Fragmenten ergibt das stärkste Signal. Deshalb wird 1/10 der eluierten DNA dieser Fraktion mit 200 ng Bam HI-gespaltener und dephosphorylierter pBR 327-Plasmid-DNA (29) ligiert. Die anschließende Transformation von E.coli RRI (30) ergibt 3000 Tetracyclin sensitive Kolonien. Von diesen Kolonien werden 1000 nach dem vorstehend erläuterten Kolonie- Hybridisierungs-Verfahren untersucht.

Danach wird Plasmid-DNA aus 25 Kolonien, die bei der Kolonie-Hybridisierung die stärksten Signale ergeben haben, wie vorstehend beschrieben isoliert und einer Dot-Blot-Analyse unterzogen.

10 der 25 untersuchten Plasmide ergeben positive Signale. Für weitere Experimente wird deshalb DNA von 6 der positive Signale ergebenden Plasmide aus 250 ml Kulturen isoliert. Mit jedem der Plasmide wird eine Eco RI- und eine Hind III-Spaltung durchgeführt und auf einem Agarose-Gel aufgetrennt.

Die DNA wird nach Southern auf Nitrocellulose fixiert und mit dem radioaktiv markierten Oligonucleotidgemisch I hybridisiert. Die Waschtemperatur wird in drei Schritten zu 1 Minute auf 52 °C erhöht. Bei dieser Temperatur hybridisiert nur noch ein 2000 bp großen Eco RI-Fragment und ein 1500 bp großes Hind III- Fragment eines Plasmids, das als pWH 1318 bezeichnet wird. Diese Fragmentgrößen stimmen mit den Ergebnissen aus der Hybridisierung mit chromosomaler Gesamt-DNA überein.

EP 0 214 548 B1

Mit dem Oligonucleotidgemisch II ergibt die Hybridisierung kein positives Signal.

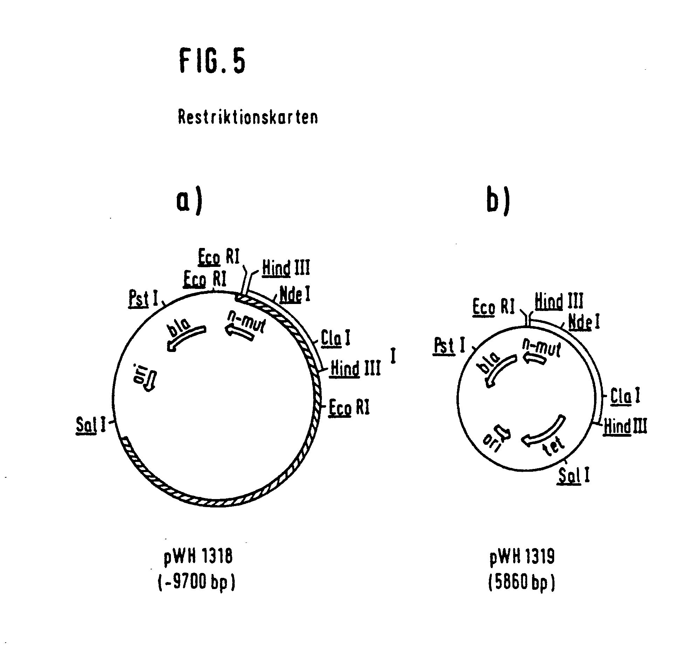

Figur 5a zeigt eine durch Spaltung mit den entsprechenden Restriktionsnucleasen ermittelte Restriktionskarte des rekombinanten Plasmids pWH 1318.

Durch Clonierung des 1500 bp großen Hind III-Fragments aus pWH 1318 in die Hind III-Spaltstelle des Plasmids pBR 322 (31) wird das rekombinante Plasmid pWH 1319 erhalten (vgl. Figur 5).

Ausgehend von der Ndel-Spaltstelle des Plasmids pWH 1319 wird die Insertion nach Maxam und Gilbert (21) ansequenziert.

Die so ermittelten DNA-Sequenz zeigt vollständige Übereinstimmung mit den ersten 18 Aminosäuren des Mutarotase-Polypeptids (vgl. Tabelle IV und Figur 4):

## Tabelle IV

| Ala | Thr | Leu | Asn | Val | | Thr | Thr | Gln | Asn | Gly | Gln | Lys | Val | Asp |
|-----|-----|-----|-----|-----|-----|-----|-----|-----|-----|-----|-----|-----|-----|-----|

GCA ACG TTA AAT GTA ←Ndel→ ACG ACT CAA AAT GGC CAA AAA GTT GAT

CA$^G_A$ AA$^C_T$ GGT CA$^G_A$ AA$^G_A$ GT

| Leu | Tyr | Thr | Met | Ser | Asn | Asn | Asn | Gly |
|-----|-----|-----|-----|-----|-----|-----|-----|-----|

CTA TAC ACC ATG AGT AAT AAC AAT GGA

Die Ergebnisse der Sequenzierung zeigen damit, daß das aus der Genbank isolierte rekombinante Plasmid pWH 1318 eine den N-terminalen Bereich des Mutarotase-Polypeptids codierende DNA-Sequenz enthält.

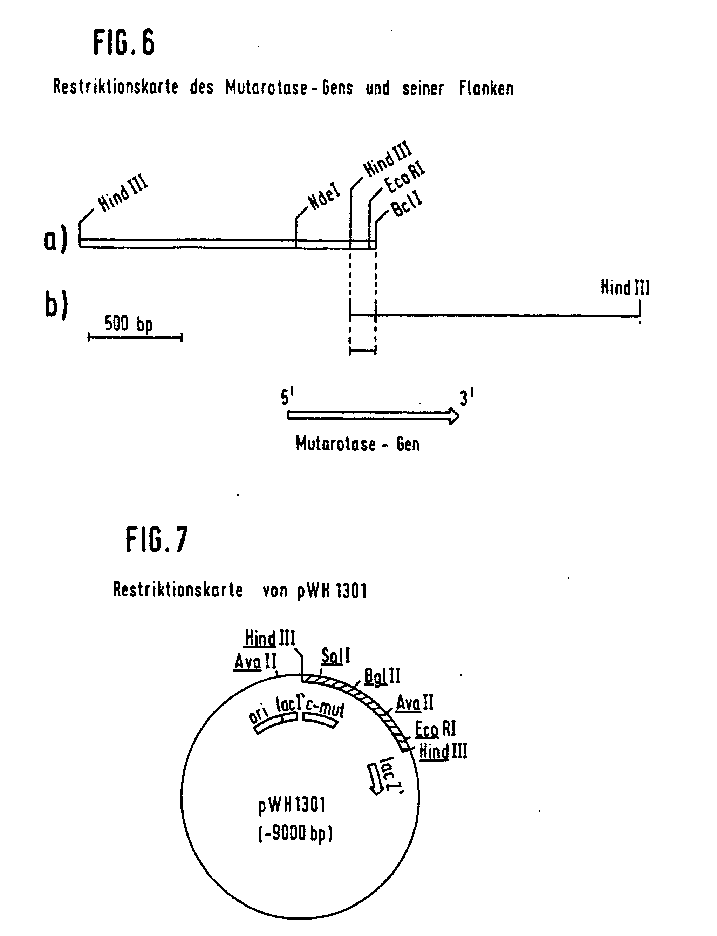

Eine den C-terminalen Bereich des Mutarotase-Polypeptids codierende DNA-Sequenz kann im Plasmid pWH 1318 nicht enthalten sein, da dieses Plasmid nicht mit dem Oligonucleotidgemisch II hybridisiert. Daher wird aus der Gesamt-DNA vom Acinetobacter calcoaceticus ein 1500 bp großes Hind III-Fragment cloniert, das mit dem Oligonucleotidgemisch II und dem etwa 125 bp langen, im rekombinanten Plasmid pWH 1318 enthaltenen Bereich zwischen der Hind III- und der Bcl I-Spaltstelle hybridisiert (vgl. Figur 6a, b).

200 µg Hind III-gespaltene chromosomale DNA werden auf einem 3prozentigen Polyacrylamidgel aufgetrennt. Die DNA im Größehbereich um 1500 bp wird eluiert. 1/4 der DNA wird wie vorstehend beschrieben auf Nitrocellulose fixiert. Die Fraktion hybridisiert sowohl mit dem Oligonucleotidgemisch II als auch mit DNA des Plasmids pWH 1318.

200 ng der Hind III-gespaltenen chromosomalen DNA aus der isolierten Fraktion werden mit 200 ng Hind III-gespaltener dephosphorylierter M13mp11-RF-DNA ligiert. Mit den erhaltenen Ligierungsprodukten werden kompetente E.coli BMH 71-18 transformiert und in Weichagar enthaltend IPTG und X-GAL ausplattiert. Die Transformation wird mehrfach wiederholt. Schließlich liegen 541 rekombinante Phagen (weiße Plaques) vor.

1 ml-Kulturen von E.coli BMH71-18 werden mit je 5 Plaques infiziert. Die gefällten Phagenüberstände werden mit dem Oligonucleotidgemisch II und mit DNA des rekombinanten Plasmids pWH 1318 hybridisiert. 2 der 131 Phagenüberstände zeigen mit beiden Sondenmolekülen positive Signale. Die Phagen werden vereinzelt in ihrer RF-Form (doppelsträngige DNA) aus einer Anzucht mit einem Volumen von 2 Litern präpariert. Eine Restriktionsanalyse der rekombinanten Phagen DNAs ergibt, daß beide positiven M13-Clone dieselbe Insertion mit der Eco RI-Spaltstelle an der erwarteten Position enthalten (Figur 7). Einer dieser beiden M13-Clone wird für die weiteren Experimente verwendet und als pWH1301 bezeichnet.

Die DNA-Sequenzierung von pWH1301 nach der M13-Methode (27) zeigt ein offenes Leseraster, das mit der Peptidsequenz des tryptischen Fragments F42 übereinstimmt (Tabelle V).

17

## Tabelle V

### Teilsequenz des M13-Clons pWH1301

```
      K    T    D    Q    P    T    V    V    N    L    T    N    H    S
     Lys  Thr  Asp  Gln  Pro  Thr  Val  Val  Asn  Leu  Thr  Asn  His  Ser
     AAA  ACT  GAT  CAG  CCT  ACA  GTC  GTC  AAC  CTT  ACC  AAC  CAC  AGT
 HindIII ——————— 86bp ——————— EcoRI ——— 30bp ——— BclI
```

```
      Y    F    N    L    S    G    A    G    N    N    P
     Tyr  Phe  Asn  Leu  Ser  Gly  Ala  Gly  Asn  Asn  Pro
     TAT  TTC  AAC  TTA  TCA  GGT  GCT  GGG  AAC  AAT  CCT
```

Die Ergebnisse der Sequenzierung bestätigten die für das Mutarotase-Gen in Figur 6a und 6b postulierte Restriktionskarte, da in der ermittelten Sequenz tatsächlich eine BclI-Spaltstelle enthalten ist (vgl. Tabelle V).

Die Clone pWH1318 bzw. pWH1319 und pWH1301 enthalten also insgesamt das vollständige Mutarotase-Gen aus Acinetobacter calcoaceticus (DSM 30008) (vgl. Figur 5, 6 und 7).

Beispiel 6

Konstruktion eines Expressionsplasmids, das die gesamte Sequenz des Mutarotase-Gens enthält

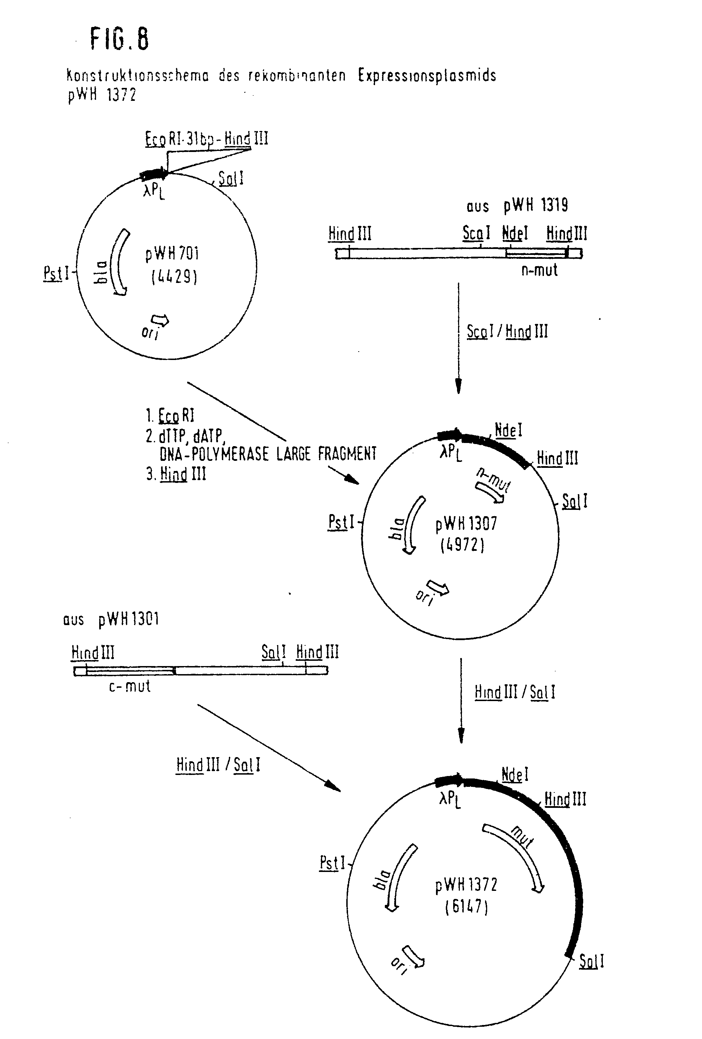

Als Expressionsplasmid wird das Plasmid pWH701 verwendet. Dabei handelt es sich um ein gut zugängliches Derivat des Plasmids pPLc236 (32), bei dem zwischen die Eco RI und die Hind III-Spaltstelle der Polylinker aus dem Plasmid pUR250 (28) eingesetzt ist. Das Plasmid pWH701 enthält den starken $\lambda$-$P_L$-Promotor (vgl. Fig. 8, schwarzer Pfeil).

Alle folgenden Clonierungsschritte werden im Wirtsbakterium E.coli W6 durchgeführt (32), in dem der $\lambda$-$P_L$-Promotor durch den $c_I$-Repressor reprimiert ist. pPLc236, der $\lambda$-$P_L$-Promotor sowie das Wirtsbakterium E.coli W6 sind auch aus der Europäischen Patentanmeldung EP 0041767 bekannt. 20 $\mu$g DNA des rekombinanten Plasmids pWH1319 werden mit den Restriktionsendonucleasen Hind III und Sca I gespalten und auf einem 5prozentigen Polyacrylamidgel elektrophoretisiert. Anschließend wird ein 574 bp großes Fragment aus dem Polyacrylamidgel eluiert.

10 $\mu$g DNA des Expressionsplasmids pWH701 werden mit der Restriktionsendonuclease Eco RI gespalten und die überstehenden Enden werden mit Klenow-Polymerase in Gegenwart von dATP und dTTP ausgefüllt. Nach Inaktivierung der Klenow-Polymerase durch 5minütiges Erhitzen des Reaktionsgemisches auf 90 °C und anschließender Äthanolfällung wird die so erhaltene DNA mit der Restriktionsendonuclease Hind III nachgespalten. Das bei dieser Spaltung anfallende 31 bp große Fragment wird durch eine Elektrophorese auf einem 3prozentigen Polyacrylamidgel abgetrennt.

200 ng auf diese Weise hergestellter Plasmid-DNA und 200 ng des wie vorstehend beschriebenen isolierten 574 bp großen DNA-Fragments werden miteinander ligiert und zur Transformation von E.coli W6 eingesetzt.

Die Plasmide der so erhaltenen Transformanten werden durch eine wie vorstehend beschriebene Schnellanalyse auf einem Agarose-Gel untersucht. Dabei zeigen die rekombinanten Expressionsplasmid-Moleküle ein höheres Molekulargewicht als die Expressionsplasmide ohne Insertion.

Eines der rekombinanten Expressionsplasmide wird für die weiteren Experimente verwendet und als pWH1307 bezeichnet. Es wird mit den Restriktionsendonucleasen Hind III sowie Sal I gespalten und wie vorstehend beschrieben aus einem Gel eluiert. Die rekombinante Phagen-DNA pWH1301 wird anschließend ebenfalls mit den Restriktionsendonucleasen Hind III und Sal I gespalten. Das dabei entstehende 1400 bp große DNA-Fragment wird wie vorstehend beschrieben aus einem Gel eluiert.

Nachfolgend wird das 1400 bp große DNA-Fragment aus pWH1301 in das große Hind III-Sal I-Fragment des Expressionsplasmids pWH1307 eingesetzt. Ligierung, Transformation und Identifizierung des gewünschten Ligierungsproduktes werden entsprechend der Konstruktion des rekombinanten Expressionsplasmids pWH1307 durchgeführt.

Das auf diese Weise erhaltene neue, erfindungsgemäße rekombinante Expressionsplasmid wird als pWH1372 bezeichnet und für die anschließende Expression verwendet.

Figur 8 zeigt ein Konstruktionsschema des rekombinanten Expressionsplasmids pWH1372.

Beispiel 7

Bestimmung der Nucleotidsequenz des Mutarotase-Gens

Die Sequenz des Gens wurde nach der Methode von Maxam und Gilbert bestimmt. Die Sequenzstrategie ist in Tab. VI dargestellt.

## Tabelle VI
## Sequenzierungsstrategie

Die vertikalen Linien der Abbildung zeigen, an welcher Restriktionsstelle die radioaktive Markierung für die Sequenzierung gesetzt wurde. Die Pfeile geben an, wie weit die Sequenz lesbar ist.

Die Sequenz zwischen der Sca I und der Hind III-Stelle wurde in pWH1318 und pWH1372 ermittelt. Die Sequenz zwischen den beiden Rsal-Stellen wurde nach Klonierung in pUR250 (28) bestimmt.

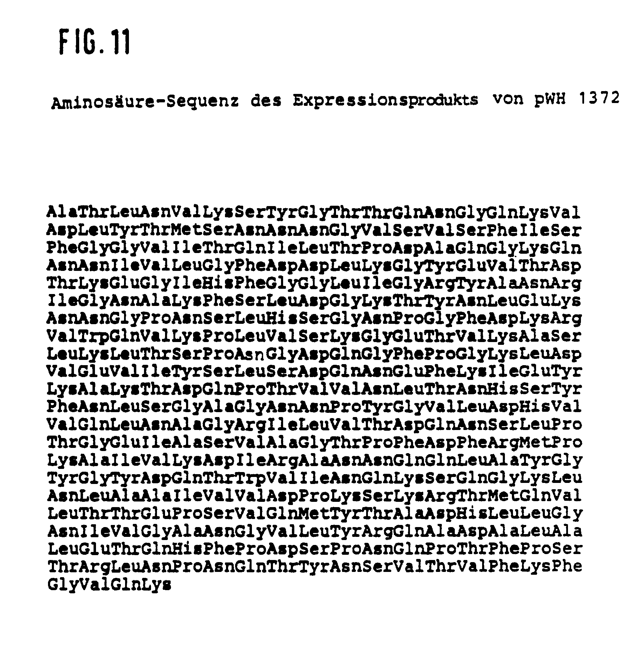

Die Nucleotidsequenz entspricht der in Figur 11 dargestellten DNA-Sequenz. Die DNA-Sequenz zeigt, daß sich vor dem durch die N-terminale Aminosäuresequenzierung ermittelten Codon eine Signalsequenz befindet. Damit ist die Aminosäuresequenz des ausgehend vom rekombinanten Expressionsplasmid pWH1372 gebildeten Polypeptids mit der biologischen Aktivität des Enzyms Mutarotase die in Figur 12 dargestellte.

Beispiel 8

Expression des Mutarotase-Gens mit dem Expressionsplasmid pWH1372 in E.coli 69

Der E. coli-Stamm Nr. 69 (E.coli K12 ΔH1) wird mit dem neuen, erfindungsgemäßen rekombinanten Expressionsplasmid pWH1372 transformiert. Dieser Wirtsbakterienstamm enthält auf seinem Chromosom einen Locus, der für den thermolabilen Repressor CI857 codiert. Daher wird in diesem Stamm die vom λ-$P_L$-Promotor gesteuerte Transkription bei 32 °C vollständig gehemmt, hingegen bei 40 °C nicht gehemmt, da bei dieser Temperatur der thermolabile Repressor CI857 in seiner inaktiven Form vorliegt.

In einem typischen Expressionsverfahren werden 200 ml LB-Medium (10 g/l Trypton, 8 g/l NaCl, 5 g/l Hefeextrakt, pH 7,8) mit 3 ml einer Übernachtkultur von E.coli 69/pWH1372 angeimpft und bei 28 °C bis zu einer Zelldichte von 0,68 OD kultiviert. Anschließend wird die Kultur in einem Wasserbadschüttler bei 40 °C weiter geschüttelt. Die Mutarotase-Aktivität wird durch den folgenden Zellaufschluß nachgewiesen.

1 ml der Zellkultur wird abzentrifugiert und die Zellen in 100 μl Aufschlußpuffer(50 mM Tris-HCl, pH 8,0, 1 mM EDTA, 5-8 % Triton) resuspendiert. Nach Zugabe von 10 μl Lysozym (25 mg/ml in 50 mM Tris-HCl, pH 8,0, 1 mM EDTA) werden die Zellen 10 Minuten bei 28 °C und 5 Minuten bei 45 °C inkubiert. Dann werden die Zellen abzentrifugiert und der Überstand auf Mutarotase-Aktivität untersucht. Die gemessene Expressionswerte von 6000 U/l Kulturmedium übertreffen die Expressionswerte des Bakterium Acinetobacter im Schüttelkolben um etwa den Faktor 60.

Vom Stamm E.coli Nr. 69/pWH1372 werden etwa 20 mg Mutarotase/l Kulturmedium gebildet. Die Ausbeute an exprimierter Mutarotase ist bei Fermentationsoptimierung im technischen Maßstab mit dem Faktor 3 bis 50 im Vergleich zum angegebenen Wert anzusetzen.

Beispiel 9

Reinigung und Analyse der in E.coli exprimierten Mutarotase

Wie aus Figur 10 ersichtlich ist, befinden sich ca. 90 der synthetisierten Mutarotase im Medium.

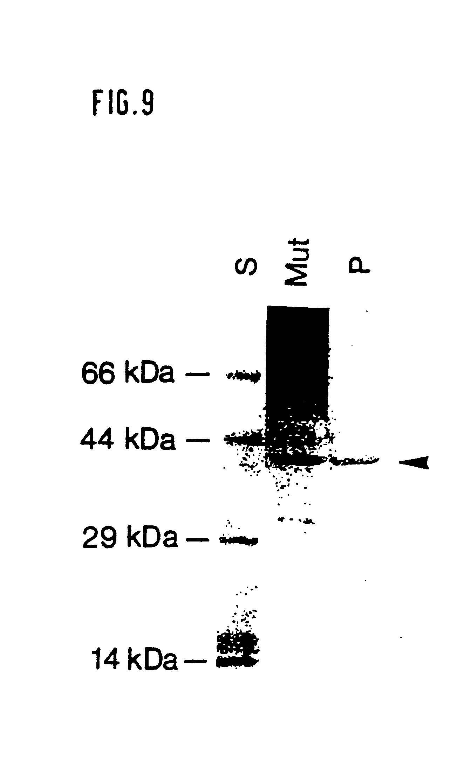

Die Proteine werden aus 1 l Medium mit 90 % Ammoniumsulfat ausgefällt, resuspendiert, gegen 20 mM $KH_2PO_4/K_2HPO_4$, pH 9,0 dialysiert und auf eine CM-Sephadexsäule (1 cm x 20 cm) aufgezogen. Es wird mit den gleichen Salzbedingungen wie in Beispiel 1 beschrieben eluiert, wobei die Mutarotase bei 50 mM $K_2HPO_4/KH_2PO_4$, pH 9,0 vom Säulenmaterial freigesetzt wird. Das Reinigungsschema ist in Tabelle VII zusammengestellt. Die Reinheit der Mutarotase vor und nach den Reinigungsschritten ist in den Spuren MUT und P des SDS-Gels (Fig. 9) zu erkennen. Die Proteinbestimmung wird dabei nach Bradford durchgeführt.

## Tabelle VII

|  | Aktivität (U) | Spez. Aktivität (U/mg) | Protein (mg) | Ausbeute (%) |
|---|---|---|---|---|
| Medium | 4700 | ND | ND | 100 |
| Ammoniumsulfatfällung | 4690 | 69 | 68 | 99 |
| Dialyse | 2800 | 54 | 52 | 60 |
| CM-Sephadex | 2400 | 200 | 12 | 52 |

Aus 1 l Medium wurden durch eine Fällung und einen säulenchromatographischen Reinigungsschritt 12 mg Protein isoliert. Die spezifische Aktivität ist um 14 % niedriger als die der aus A.calcoaceticus isolierten Mutarotase. Das aus E.coli isolierte Produkt hat ein auf einem denaturierenden Polyacrylamidgel bestimmtes Molekulargewicht von 40 kDa. Dieses Molekulargewicht stimmt mit dem aus A.calcoaceticus isoliertem Protein überein.

Die Sequenzierung der N-terminalen Aminosäuren der aus dem Medium von E.coli isolierten Mutarotase zeigte, daß die Präparation ein einheitliches Produkt geliefert hatte, dessen N-terminale Sequenz lautete:

Ala Thr Leu Asn Val Lys Ser Tyr Gly

Die N-terminalen Aminosäuren beider Produkte stimmen überein. In Position 7 befindet sich allerdings ein Serin, während die Mutarotase aus A.calcoaceticus ein Prolin an dieser Stelle aufweist.

Die in E.coli exprimierte Mutarotase gleicht in folgenden Eigenschaften der in A.calcoaceticus exprimierten Mutarotase:

1. Die spezifische Aktivität unterscheidet sich nur um 14 %.
2. Beide Proteine weisen das gleiche Molekulargewicht auf einem denaturierenden Polyacrylamidgel auf.
3. Beide Proteine binden bei 20 mM $K_2HPO_4/KH_2PO_4$, pH 9,0 an CM-Sephadex-Ionenaustauschermaterial.
4. Beide Proteine haben die gleiche Aminosäurezusammensetzung.
5. Beide Proteine haben die gleiche N-terminale Sequenz.
6. Beide Proteine passieren die innere Membran. Allerdings wird die Mutarotase nur in E.coli ins Medium freigesetzt.

Damit ist nachgewiesen, daß das gewünschte Produkt in E.coli exprimiert werden kann. Der E.coli-Stamm produziert unter vergleichbaren Bedingungen 50-100 mal mehr Mutarotase, als der ursprünglich verwendete A.calcoaceticus. Das Produkt ist aus E.coli mit weniger Reinigungsschritten darstellbar.

Literaturstellen

1. Zabeau, M. and Roberts, R. (1979) in: "Molecular Genetics" (J.H. Taylor, ed.), Academic Press, New York, Part III, pp. 1-63

2. Ehrlich, M., Cohen, S., and McDeuitto, (1978) Cell 13, 681-689

3. Broome, S. and Gilbert, W. (1978) Proc. Natl. Acad. Sci. USA 75, 2746-2749

4. Clark, L., Hitzeman, R., and Carbon, J. (1979) Methods in Enzymol. 68, 436-442

5. Laemmlie, U.K. (1970) Nature 227, 680-685

6. Bradford, M.M. (1976) Anal. Biochem. 72, 248-254

7. Edman, P. and Henschen, A. (1975) in: "Molecular Biology, Biochemistry Biophysics" (S.B. Needleman, ed.), Vol. 8, pp. 232-279, Springer Verlag, Berlin

8. Zimmermann, C.L., Appella, E., and Pisano, J.J. (1980), Anal. Biochem. 77, 569-573

9. Salnikow, J., Lehmann, A., and Wittmann-Liebold, B. (1981), Anal. Biochem. 117, 433-442

10. Laurson, R.A. (1970) Eur. J. Biochem. 26, 89-102

11. Steffens, G.J. and Buse, G. (1976) Hoppe Seyler's Z. Physiolog. Chemie 357, 1125-1137

12. Gross, E., Wittkopp, B. (1961) J. Am. Chem. Soc. 83, 1510-1514

13. Titani, K., Sagasawi, T., Resing, K., and Walsh, K.A. (1982), Anal. Biochem. 123, 408-412

14. Mattevcci, M.D. and Caruthers, M.H. (1981) J. Am. Chem. Soc. 103, 3185-3191

15. Beaucage, S.L. and Caruthers, M.H. (1981) Tetrahedron Lett. 22, 1859-1862

16. Greene, D.J. et al. (1978) Nucl. Acids Res. 5, 2373-2390

17. Helling, R.B., Goodman, H.M., and Boyer, H.W. (1974), J. Virol. 14, 1235-1244

18. Blakesley, R.W. and Wells, R.D. (1975) Nature 257, 421-422

19. Hardies, S.C., Patient, R.K., Klein, R.D., Ho, E., Reznikoff, W.S., and Wells, R.D. (1979), J. Biol. Chem 254, 5527-5534

20. Messing, J. (1983) Meth. Enzymol. 101, 20-78

21. Maxam, A. and Gilbert, W. (1980) Meth. Enzymol. 65, 499-580

22. Wallace, R.B., Schold, M., Johanson, M.J., Dembed, P., and Itakura, K. (1981), Nucleic Acids Res. 9, 3637-3656

23. Walseth, T.F. and Johnson, R.A. (1979) Biochim. Biophys. Acta 526, 11-31

24. Weinstock, R., Sweet, R., Weiss, M., Cedar, H., and Axel, R. (1978), Proc. Natl. Acad. Sci. USA 75, 1299-1303

25. Southern, E.M. (1975) J.Mol. Biol. 98, 503-517

26. Wahl, G.M., Stern, M., and Stark, G.R. (1979) Proc. Natl. Acad. Sci. USA 76, 3683-3687

27. Messing, J., Crea, R., and Seeburg, H. (1981) Nucleic Acids Res. 9, 309-321

28. Rüther, U. (1982) Nucleic Acids Res. 10, 5765-5772

29. Soberon, C., Coverrubais, L., and Bolivar, F. (1980) Gene 9, 287-305

30. Bolivar, F., Rodriquez, R.L., Greene, P.J., Betlach, M.C., Heynecker, H.L., and Boyer, N.W. (1977), Gene 2, 95-113

31. Sutcliffe, J.G. (1978) Nucleic Acids Res. 5, 2721-2728

32. Remaut, E., Staussens, P., and Fiers, W. (1981) Gene 15, 81-93

33. Bernard, H.U. and Helinski, D.R. (1979) Meth. Enzymol. 68, 482-492

34. Winnacker, E.L. (1985), Gene und Klone, VCH-Verlag

**Patentansprüche**

1. Die DNA-Sequenz

21

```
ATGAAAAAATTAGCAATTTTAGGTGTTACGGTTTATAGCTTTGCACAACT
GGCAAATGCAGCAACGTTAAATGTAAAATCATATGGTACGACTCAAAATG
GCCAAAAAGTTGATCTATACACCATGAGTAATAACAATGGAGTCTCGGTA
TCTTTTATTAGTTTTGGTGGTGTAATTACACAAATCTTGACTCCCGATGC
CCAAGGCAAACAAAATAATATCGTTTTGGGCTTTGATGACTTAAAAGGCT
ATGAAGTCACTGATACCAAGGAAGGTATTCATTTTGGCGGATTAATTGGT
CGTTATGCGAACCGGATTGGCAATGCTAAATTTAGCTTAGATGGAAAAAC
GTATAACCTCGAAAAAAATAATGGTCCGAACTCATTACATAGCGGCAATC .
CTGGTTTTGATAAACGTGTTTGGCAAGTTAAGCCCCTCGTTTCTAAAGGT
GAAACCGTTAAAGCTTCTCTTAAGTTAACCAGCCCAAATGGAGATCAAGG
TTTTCCCGGAAAATTAGATGTAGAAGTGATCTACAGTCTTTCAGATCAAA
ATGAATTCAAGATTGAATATAAAGCCAAAACTGATCAGCCTACAGTCGTC
AACCTTACCAACCACAGTTATTTCAACTTATCAGGTGCTGGGAACAATCC
TTATGGCGTGCTAGATCATGTGGTACAACTCAATGCAGGCCGTATTCTGG
TAACCGATCAAAACTCTTTACCAACAGGTGAAATTGCTTCAGTTGCAGGT
ACGCCTTTTGATTTTCGGATGCCTAAAGCAATCGTAAAAGATATTCGAGC
AAATAATCAGCAATTGGCCTATGGATATGGCTATGACCAAACTTGGGTAA
TTAATCAAAAGTCTCAAGGAAAACTCAATCTTGCAGCTATTGTGGTTGAT
CCAAAATCTAAACGGACCATGCAAGTCTTAACCACTGAACCAAGCGTCCA
AATGTATACAGCCGATCATTTATTAGGAAATATTGTTGGCGCAAATGGCG
TACTCTATCGACAAGCAGACGCACTAGCATTAGAAACACAGCATTTTCCA
GACAGCCCGAATCAACCAACTTTCCCGTCTACACGTTTAAACCCAAATCA
AACTTATAACAGTGTTACCGTATTTAAGTTTGGTGTTCAAAAA
```

die für ein Polypeptid mit der biologischen Aktivität des Enzyms Mutarotase codiert.

2. DNA-Sequenz nach Anspruch 1 oder die mit dieser hybridisiert oder durch Mutation mit dieser verwandt ist, dadurch gekennzeichnet, daß sie aus einem Genom eines Mutarotase-bildenden Mikroorganismus oder Säugers stammt und für ein Polypeptid mit der biologischen Aktivität des Enzyms Mutarotase codiert.

3. DNA-Sequenz nach Anspruch 2, dadurch gekennzeichnet, daß sie aus dem Genom eines Mikroorganismus der Gattung Acinetobacter stammt.

4. DNA-Sequenz nach Anspruch 3, dadurch gekennzeichnet, daß sie aus dem Genom eines der Stämme Acinetobacter calcoaceticus DSM 30007, DSM 30008, DSM 30010 oder DSM 30011 stammt.

5. Rekombinantes DNA-Molekül zur Clonierung, welches eine DNA-Sequenz nach einem der Ansprüche 1 bis 4 enthält.

6. Rekombinantes DNA-Molekül, dadurch gekennzeichnet, daß es eine DNA-Sequenz, nach einem der Ansprüche 1 bis 4 enthält, die funktionell mit einer Expressions-Kontroll-Sequenz verbunden ist.

7. Rekombinantes DNA-Molekül nach Anspruch 6, dadurch gekennzeichnet, daß die Expressions-Kontroll-Sequenz ein E.coli Promotor-System ist.

8. Plasmid mit der Bezeichnung pWH 1372 und der Hinterlegungsnummer DSM 3443 P und seine Mutanten.

9. Wirtsorganismus, dadurch gekennzeichnet, daß er mit mindestens einem der rekombinanten DNA-Moleküle nach einem der Ansprüche 5 bis 7 transformiert ist.

10. Wirtsorganismus nach Anspruch 9, dadurch gekennzeichnet, daß er E.coli ist.

11. Wirtsorganismus nach Anspruch 9 oder 10 mit der Bezeichnung E.coli WH 1372 und der Hinterlegungsnummer DSM 3442 und seine Mutanten.

12. Verfahren zur Herstellung eines Polypeptids mit der biologischen Aktivität des Enzyms Mutarotase durch Kultivierung eines Mikroorganismus in einem Nährmedium und Isolierung des bei der Expression gebildeten Polypeptids, dadurch gekennzeichnet, daß man als Mikroorganismus einen mit einer rekombinanten .DNA-Molekül gemäß einem der Ansprüche 5 oder 6 transformierten Wirtsorganismus einsetzt.

**Claims**

1. The DNA sequence

```
ATGAAAAAATTAGCAATTTTAGGTGTTACGGTTTATAGCTTTGCACAACT
GGCAAATGCAGCAACGTTAAATGTAAAATCATATGGTACGACTCAAAATG
GCCAAAAAGTTGATCTATACACCATGAGTAATAACAATGGAGTCTCGGTA
TCTTTTATTAGTTTTGGTGGTGTAATTACACAAATCTTGACTCCCGATGC
CCAAGGCAAACAAAATAATATCGTTTTGGGCTTTGATGACTTAAAAGGCT
ATGAAGTCACTGATACCAAGGAAGGTATTCATTTTGGCGGATTAATTGGT
CGTTATGCGAACCGGATTGGCAATGCTAAATTTAGCTTAGATGGAAAAAC
GTATAACCTCGAAAAAAATAATGGTCCGAACTCATTACATAGCGGCAATC
CTGGTTTTGATAAACGTGTTTGGCAAGTTAAGCCCCTCGTTTCTAAAGGT
GAAACCGTTAAAGCTTCTCTTAAGTTAACCAGCCCAAATGGAGATCAAGG
TTTTCCCGGAAAATTAGATGTAGAAGTGATCTACAGTCTTTCAGATCAAA
ATGAATTCAAGATTGAATATAAAGCCAAAACTGATCAGCCTACAGTCGTC
AACCTTACCAACCACAGTTATTTCAACTTATCAGGTGCTGGGAACAATCC
TTATGGCGTGCTAGATCATGTGGTACAACTCAATGCAGGCCGTATTCTGG
TAACCGATCAAAACTCTTTACCAACAGGTGAAATTGCTTCAGTTGCAGGT
ACGCCTTTTGATTTTCGGATGCCTAAAGCAATCGTAAAAGATATTCGAGC
AAATAATCAGCAATTGGCCTATGGATATGGCTATGACCAAACTTGGGTAA
TTAATCAAAAGTCTCAAGGAAAACTCAATCTTGCAGCTATTGTGGTTGAT
CCAAAATCTAAACGGACCATGCAAGTCTTAACCACTGAACCAAGCGTCCA
AATGTATACAGCCGATCATTTATTAGGAAATATTGTTGGCGCAAATGGCG
TACTCTATCGACAAGCAGACGCACTAGCATTAGAAACACAGCATTTTCCA
GACAGCCCGAATCAACCAACTTTCCCGTCTACACGTTTAAACCCAAATCA
AACTTATAACAGTGTTACCGTATTTAAGTTTGGTGTTCAAAAA
```

which codes for a polypeptide having the biological activity of the enzyme mutarotase.

2. DNA sequence according to Claim 1 or which hybridises with the latter or is related to the latter by mutation, characterised in that it is derived from the genome of a mutarotase-forming microorganism or mammal and codes for a polypeptide having the biological activity of the enzyme mutarotase.

3. DNA sequence according to Claim 2, characterised in that it is derived from the genome of a microorganism of the genus Acinetobacter.

4. DNA sequence according to Claim 3, characterised in that it is derived from the genome of one of the strains Acinetobacter calcoaceticus DSM 30007, DSM 30008, DSM 30010 or DSM 30011.

5. Recombinant DNA molecule for cloning, which contains a DNA sequence according to one of Claims 1 to 4.

6. Recombinant DNA molecule, characterised in that it contains a DNA sequence, according to one of Claims 1 to 4, which is functionally connected to an expression control sequence.

7. Recombinant DNA molecule according to Claim 6, characterised in that the expression control sequence is an E.coli promoter system.

8. Plasmid, called pWH 1372, with deposit number DSM 3443 P, and its mutants.

23

9. Host organism characterised in that it is transformed with at least one of the recombinant DNA molecules according to one of Claims 5 to 7.

10. Host organism according to Claim 9, characterised in that it is E.coli.

11. Host organism according to Claim 9 or 10, called E.coli WH 1372, with deposit number DSM 3442, and its mutants.

12. Process for the preparation of a polypeptide having the biological activity of the enzyme mutarotase by cultivation of a microorganism in a nutrient medium and isolation of the polypetide formed by expression, characterised in that the microorganism used is a host organism transformed with a recombinant DNA molecule according to one of Claims 5 or 6.

**Revendications**

1. La séquence d'ADN

```
ATGAAAAAATTAGCAATTTTAGGTGTTACGGTTTATAGCTTTGCACAACT
GGCAAATGCAGCAACGTTAAATGTAAAATCATATGGTACGACTCAAAATG
GCCAAAAAGTTGATCTATACACCATGAGTAATAACAATGGAGTCTCGGTA
TCTTTTATTAGTTTTGGTGGTGTAATTACACAAATCTTGACTCCCGATGC
CCAAGGCAAACAAAATAATATCGTTTTGGGCTTTGATGACTTAAAAGGCT,
ATGAAGTCACTGATACCAAGGAAGGTATTCATTTTGGCGGATTAATTGGT
CGTTATGCGAACCGGATTGGCAATGCTAAATTTAGCTTAGATGGAAAAAC
GTATAACCTCGAAAAAAATAATGGTCCGAACTCATTACATAGCGGCAATC
CTGGTTTTGATAAACGTGTTTGGCAAGTTAAGCCCCTCGTTTCTAAAGGT
GAAACCGTTAAAGCTTCTCTTAAGTTAACCAGCCCAAATGGAGATCAAGG
TTTTCCCGGAAAATTAGATGTAGAAGTGATCTACAGTCTTTCAGATCAAA
ATGAATTCAAGATTGAATATAAAGCCAAAACTGATCAGCCTACAGTCGTC
AACCTTACCAACCACAGTTATTTCAACTTATCAGGTGCTGGGAACAATCC
TTATGGCGTGCTAGATCATGTGGTACAACTCAATGCAGGCCGTATTCTGG,
TAACCGATCAAAACTCTTTACCAACAGGTGAAATTGCTTCAGTTGCAGGT,
ACGCCTTTTGATTTTCGGATGCCTAAAGCAATCGTAAAAGATATTCGAGC
AAATAATCAGCAATTGGCCTATGGATATGGCTATGACCAAACTTGGGTAA
TTAATCAAAAGTCTCAAGGAAAACTCAATCTTGCAGCTATTGTGGTTGAT
CCAAAATCTAAACGGACCATGCAAGTCTTAACCACTGAACCAAGCGTCCA
AATGTATACAGCCGATCATTTATTAGGAAATATTGTTGGCGCAAATGGCG
TACTCTATCGACAAGCAGACGCACTAGCATTAGAAACACAGCATTTTCCA
GACAGCCCGAATCAACCAACTTTCCCGTCTACACGTTTAAACCCAAATCA
AACTTATAACAGTGTTACCGTATTTAAGTTTGGTGTTCAAAAA
```

qui code pour un polypeptide ayant l'activité biologique de l'enzyme mutarotase.

2. Séquence d'ADN selon la revendication 1, ou qui est hybridée avec elle ou apparentée avec elle par mutation, caractérisée en ce qu'elle provent d'un génome d'un microorganisme ou mammifère formant une mutarotase et code pour un polypeptide ayant l'activité biologique de l'enzyme mutarotase.

3. Séquence d'ADN selon la revendication 2, caractérisée en ce qu'elle provient d'un génome d'un microorganisme du genre Acinetobacter.

4. Séquence d'ADN selon la revendication 3, caractérisée en ce qu'elle provient d'un génome des souches Acinetobacter calcoaceticus DSM 30007, DSM 30008, DSM 30010 ou DSM 30011.

5. Molécule d'ADN recombinant aux fins de clonage qui contient une séquence d'ADN selon l'une des revendications 1 à 4.

6. Molécule d'ADN recombinant, caractérisé en ce qu'elle contient une séquence d'ADN selon l'une des revendication 1-4 qui est fonctionnellement liée à une séquence de commande d'expression.

7. Molécule d'ADN recombinant selon la revendication 6, caractérisée en ce que la séquence de commande d'expression est un système promoteur d'E. coli.

8. Plasmide désigné par pWH1372 et par le n° de dépôt DSM 3443 P et ses mutants.

9. Organismee-hôte caractérisé en ce qu'il est transformé avec au moins une des molécules d'ADN recombinant selon l'une des revendications 5 à 7.

10. Organisme-hôte selon la revendication 9, caractérisé en ce qu'il s'agit de E. coli.

11. Organisme-hôte selon la revendication 9 ou 10 désigné par E. coli WH 1372 et le n° de dépôt DSM 3442 et ses mutants.

12. Procédé de préparation d'un polypeptide ayant l'activité biologique de l'enzyme mutarotase par culture d'un microorganisme dans un milieu nutritif et isolement du polypeptide formé dans l'expression, caractérisé en ce qu'on utilise comme microorganisme un organisme-hôte transformé avec une molécule d'ADN recombinant selon l'une des revendications 5 ou 6.

# FIG.1

Reinigung des Rohextrakts

| S | 1 | 2 | 3 | 4 | S | |
|---|---|---|---|---|---|---|
| | | | | | | — 66 kDa |
| | | | | | | — 44 kDa |
| | | | | | | — 29 kDa |
| | | | | | | — 14 kDa |

# FIG. 2

Trennung der Bromcyanfragmente

# FIG.3

Trennung der tryptischen Peptide des Mutarotase-Polypeptids

# FIG.3a

Elutionschromatogramm der ARG C-Fragmente

Sequenzierungsstrategie des Mutarotase-Polypeptids und
partielle Polypeptid-Sequenz.

```
                 ATLNVKPYGTTQNGQKVDLYTMSHNNG...................... 50

N-Terminus  ••••••••••••••••••••••••
CB 1        •••••••••••••••••••••
                            CB 2  •••••

            ................................................... 100

            ................................................... 150

            .....................TDQPTVVNLTNHSYFNLSGAGNNP... 200

                        F 42    ••••••••••••••••••••••••

            .......................................MPKAI........ 250

                              CB 3      ••••

            ...........................................TMQVLTTEPSVQMYTA 300

                                    CB 5      •••
                        F 38    •••••••••••••••••
                                    ARG 1    •••

                 DHLLGNIVGANGVLYRQADALALETQHFPDSPNQPTFPSTRLNPNQTYNS 350

CB 5        ••••••••••••••••••••••••••••••••••••••••••
F 38        ••••••••••••
ARG 1       •••••••••••••••••••                        ARG 3  •••••••••
                      ARG 2    •••••••••••••••••••••••
                 VTVFKFGVQK
ARG 3       ••••••••••
```

# FIG. 4

# FIG.5

Restriktionskarten

a)

b)

pWH 1318
(-9700 bp)

pWH 1319
(5860 bp)

# FIG. 6

Restriktionskarte des Mutarotase-Gens und seiner Flanken

500 bp

Mutarotase - Gen

# FIG. 7

Restriktionskarte von pWH 1301

pWH 1301
(~9000 bp)

# FIG. 8

Konstruktionsschema des rekombinanten Expressionsplasmids
pWH 1372

FIG.9

66 kDa —

44 kDa —

29 kDa —

14 kDa —

S    Mut    P

# FIG.10

DNA-Sequenz des Mutarotase-Gens aus Acinetobacter calcoaceticus

ATGAAAAAATTAGCAATTTTAGGTGTTACGGTTTATAGCTTTGCACAACT
GGCAAATGCAGCAACGTTAAATGTAAAATCATATGGTACGACTCAAAATG
GCCAAAAAGTTGATCTATACACCATGAGTAATAACAATGGAGTCTCGGTA
TCTTTTATTAGTTTTGGTGGTGTAATTACACAAATCTTGACTCCCGATGC
CCAAGGCAAACAAAATAATATCGTTTTGGGCTTTGATGACTTAAAAGGCT
ATGAAGTCACTGATACCAAGGAAGGTATTCATTTTGGCGGATTAATTGGT
CGTTATGCGAACCGGATTGGCAATGCTAAATTTAGCTTAGATGGAAAAAC
GTATAACCTCGAAAAAAATAATGGTCCGAACTCATTACATAGCGGCAATC
CTGGTTTTGATAAACGTGTTTGGCAAGTTAAGCCCCTCGTTTCTAAAGGT
GAAACCGTTAAAGCTTCTCTTAAGTTAACCAGCCCAAATGGAGATCAAGG
TTTTCCCGGAAAATTAGATGTAGAAGTGATCTACAGTCTTTCAGATCAAA
ATGAATTCAAGATTGAATATAAAGCCAAAACTGATCAGCCTACAGTCGTC
AACCTTACCAACCACAGTTATTTCAACTTATCAGGTGCTGGGAACAATCC
TTATGGCGTGCTAGATCATGTGGTACAACTCAATGCAGGCCGTATTCTGG
TAACCGATCAAAACTCTTTACCAACAGGTGAAATTGCTTCAGTTGCAGGT
ACGCCTTTTGATTTTCGGATGCCTAAAGCAATCGTAAAAGATATTCGAGC
AAATAATCAGCAATTGGCCTATGGATATGGCTATGACCAAACTTGGGTAA
TTAATCAAAAGTCTCAAGGAAAACTCAATCTTGCAGCTATTGTGGTTGAT
CCAAAATCTAAACGGACCATGCAAGTCTTAACCACTGAACCAAGCGTCCA
AATGTATACAGCCGATCATTTATTAGGAAATATTGTTGGCGCAAATGGCG
TACTCTATCGACAAGCAGACGCACTAGCATTAGAAACACAGCATTTTCCA
GACAGCCCGAATCAACCAACTTTCCCGTCTACACGTTTAAACCCAAATCA
AACTTATAACAGTGTTACCGTATTTAAGTTTGGTGTTCAAAAA

# FIG. 11

Aminosäure-Sequenz des Expressionsprodukts von pWH 1372

```
AlaThrLeuAsnValLysSerTyrGlyThrThrGlnAsnGlyGlnLysVal
AspLeuTyrThrMetSerAsnAsnAsnGlyValSerValSerPheIleSer
PheGlyGlyValIleThrGlnIleLeuThrProAspAlaGlnGlyLysGln
AsnAsnIleValLeuGlyPheAspAspLeuLysGlyTyrGluValThrAsp
ThrLysGluGlyIleHisPheGlyGlyLeuIleGlyArgTyrAlaAsnArg
IleGlyAsnAlaLysPheSerLeuAspGlyLysThrTyrAsnLeuGluLys
AsnAsnGlyProAsnSerLeuHisSerGlyAsnProGlyPheAspLysArg
ValTrpGlnValLysProLeuValSerLysGlyGluThrValLysAlaSer
LeuLysLeuThrSerProAsnGlyAspGlnGlyPheProGlyLysLeuAsp
ValGluValIleTyrSerLeuSerAspGlnAsnGluPheLysIleGluTyr
LysAlaLysThrAspGlnProThrValValAsnLeuThrAsnHisSerTyr
PheAsnLeuSerGlyAlaGlyAsnAsnProTyrGlyValLeuAspHisVal
ValGlnLeuAsnAlaGlyArgIleLeuValThrAspGlnAsnSerLeuPro
ThrGlyGluIleAlaSerValAlaGlyThrProPheAspPheArgMetPro
LysAlaIleValLysAspIleArgAlaAsnAsnGlnGlnLeuAlaTyrGly
TyrGlyTyrAspGlnThrTrpValIleAsnGlnLysSerGlnGlyLysLeu
AsnLeuAlaAlaIleValValAspProLysSerLysArgThrMetGlnVal
LeuThrThrGluProSerValGlnMetTyrThrAlaAspHisLeuLeuGly
AsnIleValGlyAlaAsnGlyValLeuTyrArgGlnAlaAspAlaLeuAla
LeuGluThrGlnHisPheProAspSerProAsnGlnProThrPheProSer
ThrArgLeuAsnProAsnGlnThrTyrAsnSerValThrValPheLysPhe
GlyValGlnLys
```